# EUROPEAN PATENT APPLICATION

(11) **EP 2 269 689 A1**
(43) Date of publication of application: **05.01.2011**
(21) Application number: 10075442.3
(22) Date of filing: 29.09.2006
(51) Int. Cl.: A61N 1/30

(54) **Pulsatile delivery of gonadotropin-releasing hormone from a pre-loaded integrated electrotransport patch**

(30) Priority: 30.09.2005 US 722602 P
(62) Divisional of application: 06815896.3
(71) Applicant: Vyteris Inc., Fair Lawn NJ 07410 (US)
(72) Inventor: Sharma, Ashutosh, Springfield New Jersey 07081 (US); Patel, Sonal R., Chestnut Ridge New York 10977 (US); Reddy, Vilambi N. R. K., Trichy 620-002 Tanil Nadu (IN); Kuesch, Preston, Jonesbro Maine 04648 (US); Kalia, Yogeshvar Nath, 74520 Chevrier (FR)
(74) Representative: Probert, Gareth David

(57) **Abstract**

An integrated electrode assembly structured for use in association with an electrically assisted delivery device for delivery of a composition through a membrane, said integrated electrode assembly comprising: a flexible backing; an electrode layer connected to said flexible backing, said electrode layer having at least a donor electrode and a return electrode; at least one lead extending from each of said donor electrode and said return electrode to a tab end portion of said assembly, said tab end portion being structured for electrical connection with at least on component of said electrically assisted delivery device; a donor reservoir positioned in electrical communication with said donor electrode, said donor reservoir including an amount of a composition comprising GnRH; a return reservoir positioned in electrical communication with said return electrode; at least one spline formed in said electrode layer; at least a portion of said flexible backing having a flexural rigidity less than a flexural rigidity of at least a portion of said electrode layer; wherein a ratio of a surface area of at least one of said reservoirs to a surface area of its corresponding electrode is in the range of about 1.0 to about 1.5; and wherein at least one component of said assembly in surface contact with at least one of said reservoirs has an aqueous absorption capacity less than an aqueous absorption capacity of said reservoir in surface contact with said component of said assembly.

## Description

### BACKGROUND

### Field of the Invention

The present invention generally relates to various assemblies, devices and systems structured for use in association with various electrically assisted delivery devices and systems for the delivery of gonadotropin-releasing hormone (GnRH).

### Description of the Related Art

Transdermal drug delivery systems have, in recent years, become an increasingly important means of administering drugs. Such systems offer advantages clearly not achievable by other modes of administration such as introduction of the drug through the gastro-intestinal tract or punctures in the skin, to name a few.

There are two types of transdermal drug delivery systems, "passive" and "active." Passive systems deliver a medicament through the skin of the user unaided, an example of which would involve the application of a topical anesthetic to provide localized relief, as disclosed in U.S. Patent No. 3,814,095. Active systems, on the other hand, use external force to facilitate delivery of a medicament through a patient's skin. Examples of active systems include ultrasound, electroporation, and/or iontophoresis.

Iontophoretic delivery of a medicament is accomplished by application of a voltage to a medicament-loaded reservoir-electrode, sufficient to maintain a current between the medicament-loaded reservoir-electrode and a return reservoir electrode (another electrode) applied to a patient's skin so that the desired medicament is delivered to the patient in ionic form.

Conventional iontophoretic devices, such as those described in U.S. Patent Nos. 4,820,263; 4,927,408; and 5,084,008, the disclosures of which are hereby incorporated by reference, deliver a drug transdermally by iontophoresis. These devices basically consist of two electrodes - an anode and a cathode. In a typical iontophoretic device, electric current is driven from an external power supply. In a device for delivering drug from an anode, positively charged drug is delivered into the skin at the anode, with the cathode completing the electrical circuit. Likewise, in a system for delivering drug from a cathode, negatively charged drug is delivered into the skin at the cathode, with the anode completing the electrical circuit. Accordingly, there has been considerable interest in iontophoresis to perform delivery of drugs for a variety of purposes. One example is the delivery of lidocaine, a common topical, local anesthetic.

The therapeutic objective of gonadotropin-releasing hormone ("GnRH") and analogs thereof includes, without limitation, management of amenorrhoea and infertility related to hypogonadotrophic hypogonadism. Gonadorelin (gonadrelin (HCl), commercially available in the United States under the trade name FACTREL^{®} (a registered trademark of Wyeth-Ayerst Laboratories, Madison, New Jersey) or gonadorelin acetate, commercially available in Canada under the trade name LUTREPULSE^{®} (a registered trademark of Ferring Pharmaceuticals of Suffern, New York)) is a synthetic decapeptide that has the same amino acid sequence as endogenous GnRH synthesized in the human hypothalamus and in various neurons terminating in the hypothalamus. Its pharmacological and toxicological profile is therefore identical to that of endogenous GnRH. Other synonyms of GnRH include: luteinizing hormone-releasing hormone (LHRH), luteinizing hormone-releasing factor dihydrochloride (gonadorelin hydrochloride), luteinizing hormone-releasing factor diacetate tetrahydrate (gonadorelin acetate), and luteinizing hormone-/follicle-stimulating hormone-releasing hormone (LH/FSH-RH). GnRH causes the pituitary gland to release other hormones (luteinizing hormone (LH) and follicle-stimulating hormone (FSH)). LH and FSH control development in children and fertility in adults. This treatment involves receiving intermittent pulses of GnRH ideally 60-120 minutes apart. This dose regimen typically is implemented by use of a subcutaneous infusion pump. This requires a surgical procedure to implant and remove the pump and the overall cumbersome nature of the therapy has limited the use of the pump system.

U.S. Patent Nos. 5,013,293; 5,312,325; 5,328,454; 5,336,168; and 5,372,579 disclose pulsatile transdermal drug delivery, including delivery of GnRH. These patents disclose delivery of GnRH according to natural rhythms to induce gonadotropin release. These patents also disclose that administration of GnRH in a steady-state mode or at an increased frequency from the natural frequency extinguishes gonadotrophic secretion. The present disclosure presents an improvement over this prior art and novel features related to the reservoir electrode, drug concentration and profiles attained. The improvement is demonstrated by actual *in vivo* delivery with the electrically assisted delivery device disclosed herein.

We have determined that an iontophoretic system can achieve the needed pulsatile dosing in a discrete, non-invasive delivery system that will allow the precise delivery of GnRH and maintain a record of dosage and time relationship.

Studies were designed to determine if GnRH could be delivered by transdermal electrotransport with a pulsatile delivery profile similar to that produced by the FACTREL^{®} intravenous (IV) pump. The pump delivers between 2.5 and 20 µg/pulse with a pulse period of 1 minute and frequency of 90 minutes. An alternative target for the studies was to simulate subcutaneous (SC) delivery, which is also efficacious in the higher dose range, ∼20 µg/pulse. We have determined that GnRH can be delivered by electrotransport. We have determined the effect of drug concentration and pulse duration, and we have determined reproducibility.

Experiments have verified that pulsatile transdermal iontophoresis reproduces pulsed secretion of GnRH and compares favorably with subcutaneous injection profiles. Shorter duration pulses (8 x 5 min) can provide a sharper plasma profile. Plasma GnRH levels were relatively independent of donor drug concentration (10-50 mg/mL) at the 8 min pulse duration but showed some dependence at the 5 minute pulse duration. Patch formulation remained stable from fabrication through the duration of use. Experiments show a ramping effect for the first two pulses, where the plasma levels of GnRH are relatively lower. This can be overcome through increasing the current density for those two pulses. The current delivered was well within acceptable limits and caused minimal to no irritation. Finally, GnRH delivery over eight five-minute pulses was reproducible.

Conventional iontophoretic devices are unable to efficiently and effectively deliver a composition, such as GnRH, through a membrane, such as skin. Improved features, such as various structural, physical, mechanical, electrical, electrochemical, and/or electromechanical elements, are required to enhance the performance of iontophoretic devices.

### SUMMARY

In various embodiments of the present invention, an integrated electrode assembly structured for use in association with an electrically assisted delivery device for delivery of a composition of GnRH through a membrane is provided. In various embodiments, the integrated electrode assembly includes a flexible backing; an electrode layer connected to the flexible backing, the electrode layer having at least a donor electrode and a return electrode; at least one lead extending from each of the donor electrode and the return electrode to a tab end portion of the assembly, the tab end portion being structured for electrical connection with at least one component of the electrically assisted delivery device; a donor reservoir positioned in electrical communication with the donor electrode, the donor reservoir including an amount of the composition; and a return reservoir positioned in electrical communication with the return electrode.

In addition, embodiments of the present invention may include at least one of the following features: an insulating dielectric coating positioned adjacent to at least a portion of at least one of the electrodes and the leads; at least one spline formed in the electrode layer; a tab stiffener connected to the tab end portion; a tab slit formed in the tab end portion; a sensor trace positioned on the tab end portion; a release cover having a donor portion structured to cover the donor reservoir and a return portion structured to cover the return reservoir; at least a portion of the flexible backing having a flexural rigidity less than a flexural rigidity of at least a portion of the electrode layer; a shortest distance between a surface area of an assembly including the donor electrode and the donor reservoir and a surface area of an assembly including the return electrode and the return reservoir being sized to provide a substantially uniform path of delivery for the composition through the membrane; a surface area of an assembly including the donor electrode and the donor reservoir is greater than a surface area of an assembly including the return electrode and the return reservoir; a ratio of a surface area of at least one of the reservoirs to a surface area of its corresponding electrode is in the range of about 1.0 to 1.5; a footprint area of the assembly is in the range of about 5 cm² to 100 cm²; a ratio of a total surface area of the electrodes to a total footprint area of the assembly is in the range of about 0.1 to 0.7; a ratio of a surface area of the donor electrode to a surface area of the return electrode is in the range of about 0.1 to 5.0; a ratio of a thickness of the donor reservoir to a thickness of the return reservoir is in the range of about 0.1 to 2.0; at least one component of the assembly in electrical communication with at least one of the reservoirs has an aqueous absorption capacity less than an aqueous absorption capacity of the reservoir in surface contact with the component of the assembly; a slit formed in the flexible backing in an area located between the donor electrode and the return electrode; at least one non-adhesive tab extending from the flexible backing; a gap formed between a portion of a layer of transfer adhesive deposited on the electrode layer and a portion of a tab stiffener connected to the tab end portion; at least one tactile sensation aid formed in the tab end portion; at least one indicium formed on at least a portion of the assembly; a minimum width of a portion of a layer of transfer adhesive deposited on the electrode layer adjacent to at least one of the donor electrode and the return electrode is in the range of at least about 0.953 cm (0.375 inches); or, a minimum tab length associated with the tab end portion is in the range of at least about 3.81 cm (1.50 inches).

Another non-limiting embodiment of the present disclosure provides a method for administering a composition through a membrane. The method comprises attaching to the membrane an integrated electrode assembly structured for use in association with an electrically assisted delivery device for delivery of a composition through a membrane. The integrated electrode assembly is described above and herein below. The method further involves applying an electrical charge of about 40 mA·min to about 100 mA·min for the electrically assisted delivery device.

### BRIEF DESCRIPTION OF THE DRAWINGS

Figure 1 (prior art) shows schematically an electrically assisted drug delivery system including an anode assembly, a cathode assembly and a controller/power supply.
Figure 2 shows an exploded isometric view of various aspects of an integrated electrode assembly provided in accordance with the present invention.
Figure 3 shows an exploded isometric view of various aspects of an integrated electrode assembly release cover provided in accordance with the present invention.
Figure 4 shows an elevated view of various aspects of an integrated electrode assembly provided in accordance with the present invention.
Figure 5A includes an exploded isometric view illustrating various aspects of the interconnection of an integrated electrode assembly provided in accordance with the present invention with components of an electrically assisted delivery device.
Figure 5B shows a schematic representation of the interaction between a portion of an integrated electrode assembly provided in accordance with the present invention and components of an electrically assisted delivery device.
Figure 5C illustrates a schematic representation of the interaction between a portion of an integrated electrode assembly provided in accordance with the present invention and components of an electrically assisted delivery device
Figure 6 includes a schematic elevated view of various aspects of an integrated electrode assembly provided in accordance with the present invention.
Figures 6B and 6C show cross-sectional views illustrating aspects of the electrode assembly of Figure 6.
Figure 7 includes a schematic elevated view of various aspects of an integrated electrode assembly provided in accordance with the present invention.
Figure 7A includes a cross-sectional view of the release cover of Fig. 7.
Figure 8 includes a schematic that illustrates the effect of electrode geometry and spacing on the delivery paths of a composition through a membrane.
Figure 9 includes a schematic that illustrates the effect of electrode geometry and spacing on the delivery paths of a composition through a membrane.
Figure 10 shows a cross-sectional view of a schematic un-loaded electrode assembly in contact with a loading solution.
Figure 11 is a cut-away view of a package including an electrode assembly release cover structured in accordance with the present invention.
Figures 12-16 show plots of plasma concentration of GnRH versus time for Examples 1, 2, and 3.
Figure 17 is a schematic of a rectangular pulsatile delivery profile.

### DETAILED DESCRIPTION

The use of numerical values in the various ranges specified in this application, unless expressly indicated otherwise, are stated as approximations as though the minimum and maximum values within the stated ranges were both preceded by the word "about." In this manner, slight variations above and below the stated ranges can be used to achieve substantially the same results as values within the ranges. Also, the disclosure of these ranges is intended as a continuous range including every value between the minimum and maximum values.

Unless otherwise specified, embodiments of the present invention are employed under "normal use" conditions, which refer to use within standard operating parameters for those embodiments. During operation of various embodiments described herein, a deviation from the target of one or more parameters of about 10% or less for an iontophoretic device under "normal use" is considered an adequate excursion for purposes of the present invention.

The terms "unloaded" or "unloaded reservoir," are necessarily defined by the process of loading a reservoir. In the loading process, a drug or other compound or composition if absorbed, adsorbed and/or diffused into a reservoir to reach a final content or concentration of the compound or composition. An unloaded reservoir is a reservoir that lacks that compound or composition in its final content or concentration. In one example, the unloaded drug reservoir is a hydrogel, as described in further detail below, which includes water and a salt. One or more additional ingredients may be included in the unloaded reservoir. Typically, active ingredients are not present in the unloaded gel reservoir. Other additional, typically non-ionic ingredients, such as preservatives, may be included in the unloaded reservoir. Although the salt may be one of many salts, including alkaline metal halide salts, the salt typically is sodium chloride. Other halide salts such as, without limitation, KCl or LiCl might be equal to NaCl in terms of functionality, but may not be preferred. Use of halide salts to prevent electrode corrosion is disclosed in U.S. Patent Nos. 6,629,968 and 6,635,045 both of which are incorporated herein by reference in their entireties.

The term "electrically assisted delivery" refers to the facilitation of the transfer of any compound through a membrane, such as, without limitation, skin, mucous membranes and nails, by the application of an electric potential across that membrane. "Electrically assisted delivery" is intended to include, without limitation, iontophoretic, electrophoretic and electroendosmotic delivery methods. By "active ingredient," it is meant, without limitation, drugs, active agents, therapeutic compounds, medicaments, and any other compound capable of eliciting any pharmacological effect in the recipient that is capable of transfer by electrically assisted delivery methods.

The term "GnRH", unless otherwise specified, refers to any water-soluble, ionizable form of GnRH, including free base, salts, or derivatives, homologs, or analogs thereof. For example, as is described in the specification and in the Examples herein, "GnRH" refers to GnRH hydrochloride (HCl); GnRH acetate, commercially available as LUTREPULSE^{®} or FACTREL^{®}, respectively, among other names; or mixtures thereof.

In other non-limiting embodiments of the present disclosure, other possible homologs, derivatives, or analogues of GnRH that may be used in place of, or in addition to GnRH, include other GnRH agonists, including peptides that produce the same pharmacological effect and may have a longer half-life, such as, for example, Buserelin, Deslorelin, Goserelin, Histrelin, Leuprolide (Leuprorelin), Nafarelin, and Triptorelin.

As applied to various embodiments of electrically assisted delivery devices described herein, the term "integrated" as used in connection with a device indicates that at least two electrodes are associated with a common structural element of the device. For example, and without limitation, a transdermal patch of an iontophoretic device may include both a cathode and an anode "integrated" therein, i.e., the cathode and anode are attached to a common backing.

As applied to various embodiments of electrically assisted delivery devices described herein, a "flexible" material or structural component is generally compliant and conformable to a variety of membrane surface area configurations and a "stiff" material or structural component is generally not compliant and not conformable to a variety of membrane surface area configurations. In addition, a "flexible" material or component possesses a lower flexural rigidity in comparison to a "stiff' material or structural component having a higher flexural rigidity. For example and without limitation, a flexible material when used as a backing for an integrated patch can substantially conform over the shape of a patient's forearm or inside elbow, whereas a comparatively "stiff" material would not substantially conform in the same use as a backing.

As applied herein, the term "transfer absorbent" includes any media structured to retain therein a fluid or fluids on an at least temporary basis and to release the retained fluids to another medium such as a hydrogel reservoir, for example. Examples of "transfer absorbents" that may be employed herein include, without liinitation, non-woven fabrics and open-cell sponges and foams.

### Iontophoretic Device

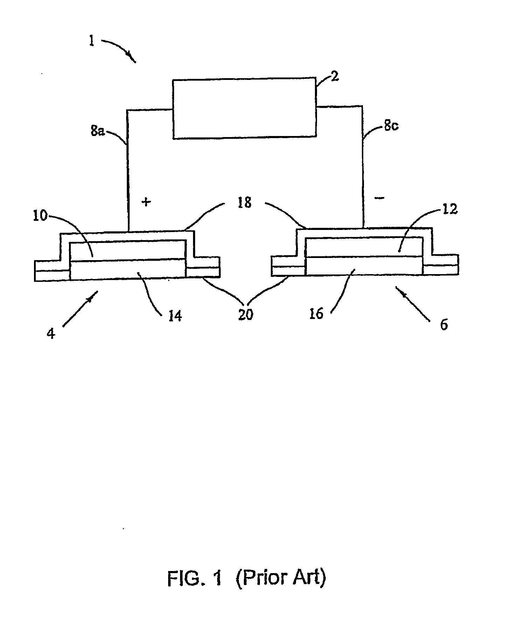

Figure 1 depicts schematically a typical electrically assisted drug delivery apparatus **1**. The apparatus **1** includes an electrical power supply/controller **2**, an anode electrode assembly **4** and a cathode electrode assembly **6**. Anode electrode assembly **4** and cathode electrode assembly **6** are connected electrically to the power supply/controller **2** by conductive leads **8a** and **8c** (respectively). The anode electrode assembly **4** includes an anode **10** and the cathode electrode assembly **6** includes a cathode **12**. The anode **10** and the cathode **12** are both in electrical contact with the leads **8a, 8c**. The anode electrode assembly **4** further includes an anode reservoir **14**, while the cathode electrode assembly **6** further includes a cathode reservoir **16**. Both the anode electrode assembly **4** and the cathode electrode assembly **6** include a backing **18** to which a pressure sensitive adhesive 20 is applied in order to affix the electrode assemblies **4**, **6** to a membrane (e.g., skin of a patient), to establish electrical contact for the reservoirs **14, 16** with the membrane. Optionally, the reservoirs **14, 16** may be at least partially covered with the pressure sensitive adhesive **20.**

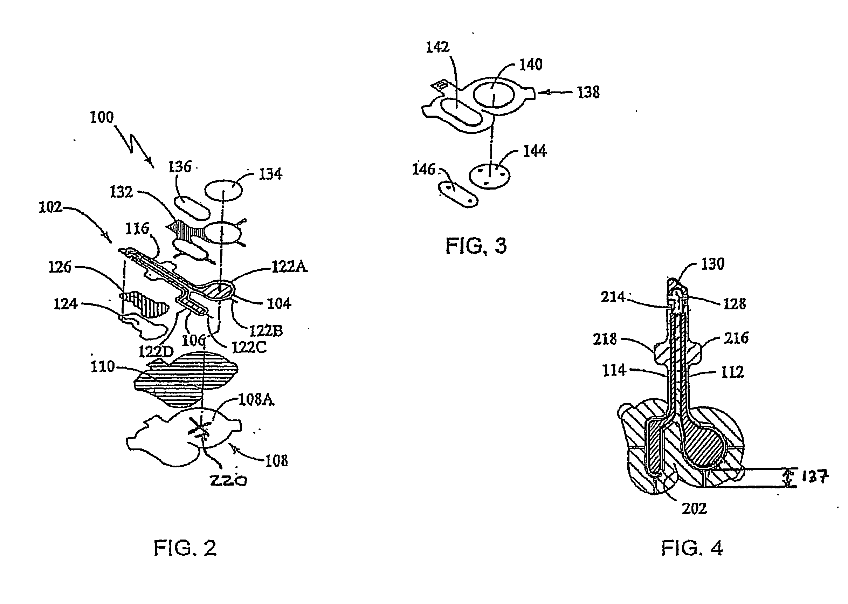

Figures 2 through 10 illustrate various aspects of an integrated electrode assembly 100 of the present invention structured for use with an electrically assisted delivery device, for example, for delivery of a composition, such as, for example, GnRH, through a membrane. A printed electrode layer **102** including two electrodes (an anode **104** and a cathode **106)** is connected to a flexible backing **108** by a layer of flexible transfer adhesive **110** positioned between the printed electrode layer **102** and the flexible backing **108**. One or more leads **112, 114** may extend from the anode **104** and/or cathode **106** to a tab end portion **116** of the printed electrode layer **102**. In various aspects, an insulating dielectric coating **118** may be deposited on and/or adjacent to at least a portion of one or more of the electrodes **104, 106** and/or the leads **112, 114.** The dielectric coating **118** may serve to strengthen or bolster the physical integrity of the printed electrode layer **102**; to reduce point source concentrations of current passing through the leads **112, 114** and/or the electrodes **104, 106;** and/or to resist creating an undesired short circuit path between portions of the anode **104** and its associated lead **112** and portions of the cathode 106 and its associated lead **114**.

In other aspects, one or more splines **122 (122A, 122B**, **122C**, **122D**) may be formed to extend from various portions of the printed electrode layer **102**, as shown. It can be seen that at least one advantage of the splines **122** is to facilitate manufacturability (e.g., diecutting of the electrode layer **102**) and construction of the printed electrode layer 102 for use in the assembly **100**. The splines **122** may also help to resist undesired vacuum formation when a release cover (see discussion hereafter) is positioned in connection with construction or use of the assembly **100.**

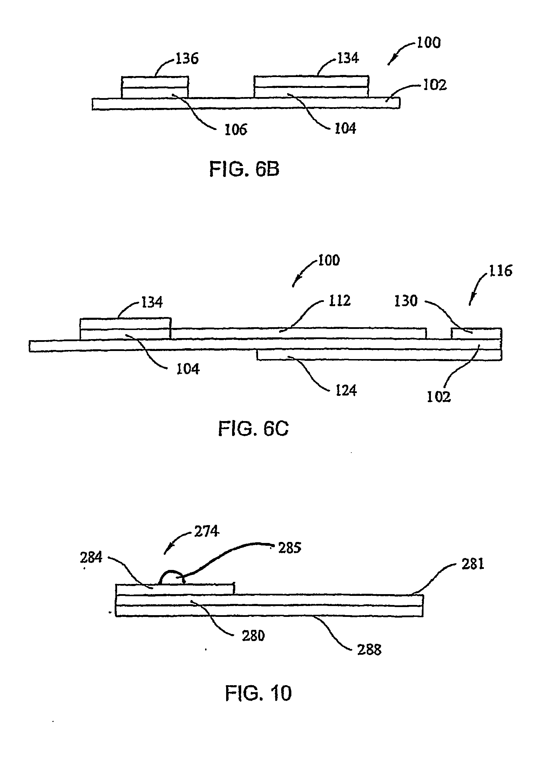

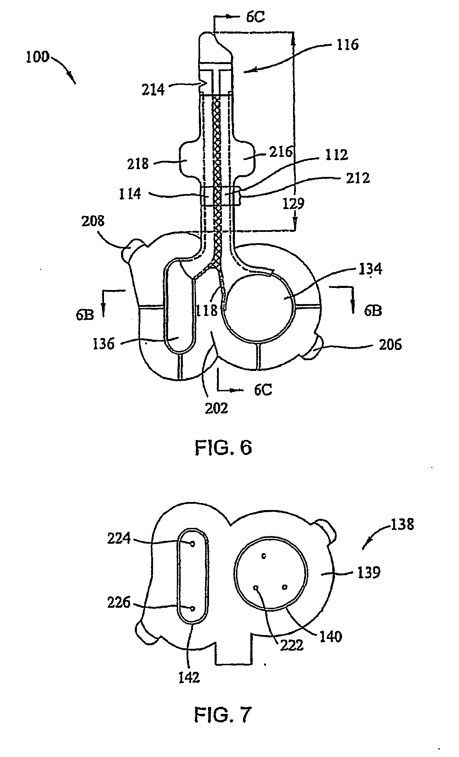

In other embodiments of the present invention, a tab stiffener **124** is connected to the tab end portion **116** of the printed electrode layer **102** by a layer of adhesive 126 positioned between the tab stiffener **124** and the tab end portion **116.** In various embodiments, a tab slit **128** may be formed in the tab end portion **116** of the assembly **100** (as shown more particularly in Figures 2 and 4). The tab slit **128** may be formed to extend through the tab stiffener **124** and the layer of adhesive **126**. In other embodiments, a minimum tab length **129** (as shown particularly in Figure 6) as structured in association with the tab end portion **116** may be in the range of at least about 1.5 inches.

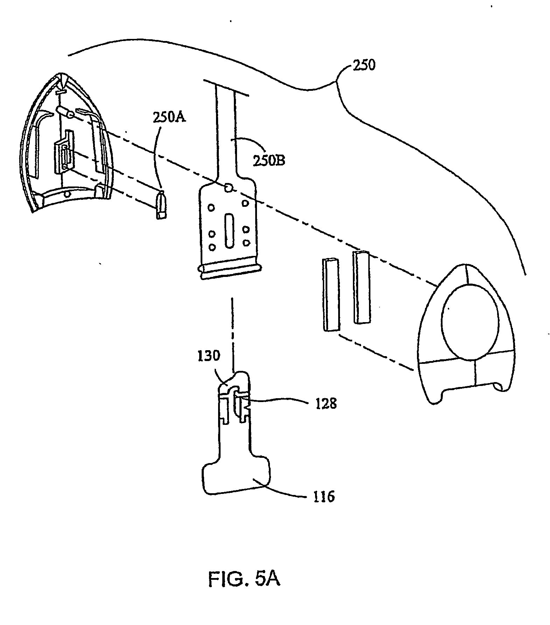

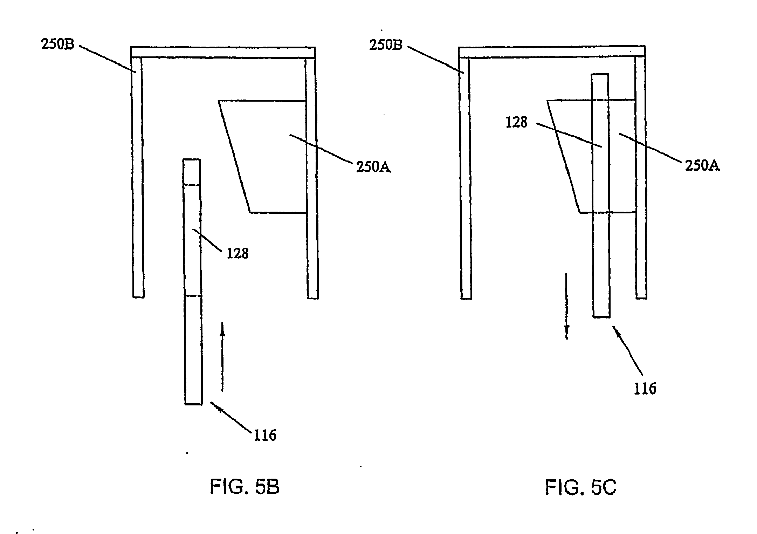

With reference to Figures 5A - 5C, the tab end portion **116** may be structured to be mechanically or electrically operatively associated with one or more components of an electrically assisted drug delivery device such as a knife edge **250A** of a connector assembly **250,** for example. As shown schematically in Figures 5B and 5C, once the tab end portion 116 is inserted into a flexible circuit connector **250B** of the connector assembly **250,** the tab slit 128 of the tab end portion **116** may be structured to receive therein the knife edge **250A.** It can be appreciated that the interaction between the knife edge **250A** and the tab slit **128** may serve as a tactile sensation aid for a user manually inserting the tab end portion 116 into the flexible circuit connector **250B** of the connector assembly **250**. In addition, the knife edge **250A** may be structured, upon removal of the tab end portion **116** from the connector assembly **250**, to cut or otherwise disable one or more electrical contact portions positioned on the tab end portion **116,** such as a sensor trace **130,** for example. It can be seen that this disablement of the electrical contact portions may reduce the likelihood that unintended future uses of the assembly **100** will occur after an initial use of the assembly **100** and the connector assembly **250** for delivery of a composition through a membrane, for example.

In other aspects, a layer of transfer adhesive **110** may be positioned in communication with the printed electrode layer **102** to facilitate adherence and/or removal of the assembly **100** from a membrane, for example, during operation of an electrically assisted delivery device that includes the assembly **100.** Optionally a second adhesive layer **132** may be positioned on the electrode layer to peripherally surround the printed electrode layer **102** and to further facilitate adherence and/or removal of the assembly **100** from a membrane. As shown in Figure 2, a first hydrogel reservoir **134** is positioned for electrical communication with the anode **104** of the printed electrode layer **102** and a second hydrogel reservoir **136** is positioned for electrical communication with the cathode **106** of the printed electrode layer **102.** In other aspects, although a hydrogel may be preferred in many instances, any aqueous conductive media containing a salt, including NaCl, for example, may be utilized as the cathode **106.**

As shown in Figure 3, a release cover **138** includes an anode-donor portion **140** and a cathode-return portion **142.** The anode-donor portion **140** is structured to receive therein a donor transfer absorbent **144** suitably configured/sized for placement within the anode-donor portion **140**. Likewise, the cathode-return portion **142** is structured to receive therein a return transfer absorbent **146** suitably configured/sized for placement within the cathode-return portion **142**. The transfer absorbents **144, 146** may be attached to their respective portions **140, 142** by a suitable method or apparatus, such as by use of one or more spot welds, for example. In construction of the assembly **100,** it can be seen that the release cover **138** is structured for surface contact with the flexible transfer adhesive layer **110** such that the donor transfer absorbent **144** establishes contact with the hydrogel reservoir **134** associated with the anode **104** and the return transfer absorbent **146** establishes contact with the hydrogel reservoir **136** associated with the cathode **106.**

In various embodiments, the integrated assembly **100** may include a first reservoir-electrode assembly (including the reservoir **134** and the anode **104**) charged with a medicament, for example GnRH (HCl) and/or GnRH (acetate) or homolog, derivative, or analog thereof, that may function as a donor assembly and a second reservoir-electrode assembly (including the reservoir **136** and the cathode **106**) that may function as a return assembly. The assembly **100** includes the reservoir-electrode **104** and the reservoir-electrode **106** mounted on an electrode assembly securement portion **108A** of the flexible backing **108.** The assembly **100** includes two electrodes, an anode **104** and a cathode **106**, each having an electrode surface and an operatively associated electrode trace or lead **112** and **114**, respectively. The electrodes **104,106** and the electrode traces **112,114** may be formed as a thin film deposited onto the electrode layer **102** by use of a conductive ink, for example. The conductive ink may include Ag and Ag/AgCl, for example, in a suitable binder material, and the conductive ink may have the same composition for both the electrodes **104**, **106** and the electrode traces **112, 114.** A substrate thickness for the conductive ink may be in the range of about 0.05 mm (0.002 inches) to about 0.18 mm (0.007 inches). In other aspects of certain non-limiting embodiments, the specific capacity of the conductive ink for Ag/AgCl electrochemistry is preferably in the range of about 2 to about 120 mA·min/cm², or more preferably in the range of about 5 to about 20 miA·min/cm². In another embodiment, the specific capacity of the conductive ink for Ag/AgCl electrochemistry is most preferably in the range of about 20 to about 40 mA·min/cm². In various aspects, the conductive ink may comprise a printed conductive ink. The electrodes **104, 106** and the electrode traces **112, 114** may be formed in the electrode layer **102** to comprise a stiff portion of the assembly **100.**

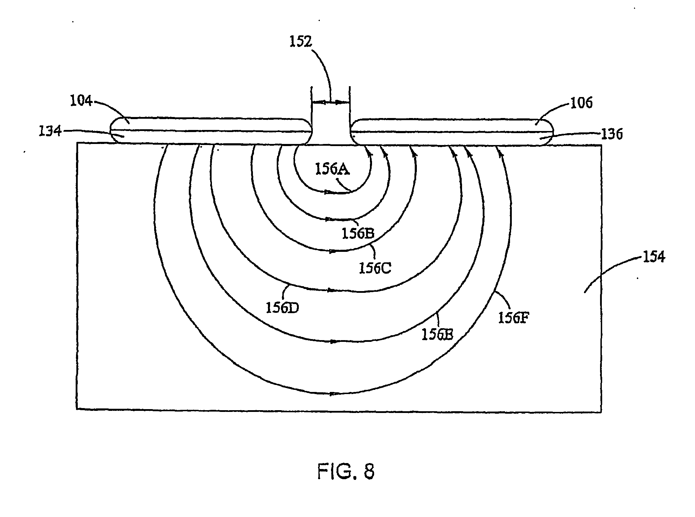

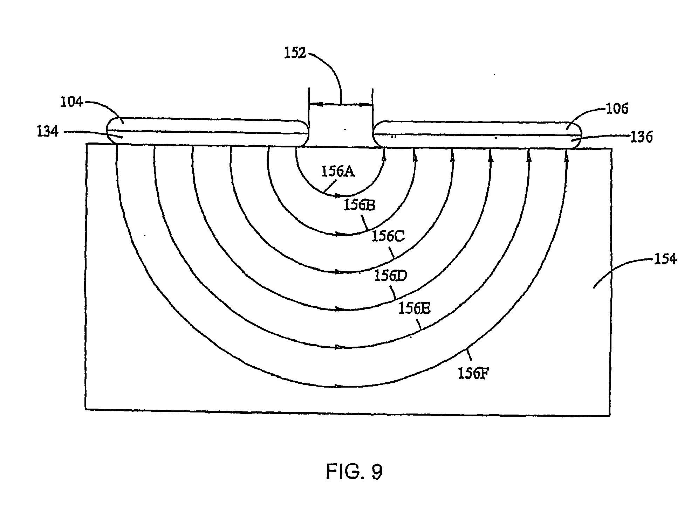

In various embodiments of the present invention, a shortest distance **152** between a surface area of the anode **104** / reservoir **134** assembly and a surface area of the cathode **106** / reservoir **136** assembly may be in the range of at least about 0.064 cm (0.25 inches). Referring now to Figure 8, for example, it can be seen that inappropriate selection of the distance **152,** the geometric configuration of the electrodes **104, 106** (e.g., thickness, width, total surface area, and others), and/or a combination of other factors may result in a substantially non-uniform delivery of a composition between the electrodes through a membrane **154** during operation of the assembly **100.** As shown, the delivery of the composition through the membrane is shown schematically by composition delivery paths **156A - 156F.** In contrast, as shown in Figure 9, appropriate selection of the distance **152,** the geometric configuration of the electrodes **104,106** (e.g., thickness, width, total surface area, and others), and/or a combination of other factors may result in a substantially uniform delivery of a composition between the electrodes through a membrane **154** as shown by delivery paths **156A - 156F.** It can be seen that the inventors have recognized the problem of delivering a composition through a membrane that may include scar tissue, for example, or another variation in the density of the membrane that may adversely impact the effectiveness and uniformity of delivery of the composition between the electrodes of a device, for example.

In accordance with discussion above, the electrodes **104, 106** may each be mounted with bibulous reservoirs **134, 136** (respectively) formed from a cross-linked polymeric material such as cross-linked poly(vinylpyrrolidone) hydrogel, for example, including a substantially uniform concentration of a salt, for example. The reservoirs **134, 136** may also include one or more reinforcements, such as a low basis weight non-woven scrim, for example, to provide shape retention to the hydrogels. The reservoirs **134, 136** each may have adhesive and cohesive properties that provide for releasable adherence to an applied area of a membrane (e.g., the skin of a patient). In various embodiments, the strength of an adhesive bond formed between portions of the assembly **100** and the application area or areas of the membrane is less than the strength of an adhesive bond formed between the membrane and the reservoirs **134, 136.** These adhesive and cohesive properties of the reservoirs **134, 136** have the effect that when the assembly **100** is removed from an applied area of a membrane, a substantial amount of adhesive residue, for example, does not remain on the membrane. These properties also permit the reservoirs **134, 136** to remain substantially in electrical communication with their respective electrodes **104, 136** and the flexible backing **108** to remain substantially in electrical communication with the printed electrode layer **102.**

Portions of the assembly **100,** as provided in accordance with embodiments of the present invention, may be structured to exhibit flexibility or low flexural rigidity in multiple directions along the structure of the device **100.** Working against flexibility of the device **100**, however, may be the construction of the comparatively stiffer electrode layer **102,** which may include a material such as print-treated polyethylene terephthalate (PET), for example, as a substrate. PET is a relatively strong material exhibiting high tensile strength in both the machine and transverse directions and having a flexural rigidity, G=E*δⁿ, which is a function of modulus of elasticity (E) and a power of the thickness (δ) of the material. By way of a hypothetical counter-example, if a substance such as MYLAR™, for example, were to be used for both the electrode layer **102** and the flexible backing **108**, at least two problems would be presented: the assembly **100** would be too inflexible to fully or effectively adhere to a site of treatment on a membrane; and for removal from the membrane once treatment is completed, the assembly **100** would require a relatively high level of force, due to the strength of the flexible backing **108,** to remove the assembly **100.**

Embodiments of the present invention provide the flexible backing **108** around the periphery of the stiff electrode layer **102.** In certain aspects, a relatively thin and highly compliant flexible backing composed of about 0.010 cm (0.004 inch) ethyl vinyl acetate (EVA), for example, may be used for the flexible backing **108.** This configuration offers a flexible and compliant assembly **100** in multiple planar directions, permitting the assembly **100** to conform to the contour of a variety of membranes and surfaces. In addition, a pressure sensitive adhesive (e.g., polyisobutylene (PIB)) may be applied as the transfer adhesive layer **110** to mitigate a potential decrease in flexibility of the flexible backing **108.** It can be seen that, in various embodiments, devices constructed in accordance with the present invention permit a degree of motion and flexure during treatment without disrupting the function of the assembly **100.** The assembly **100** therefore exhibits low flexural rigidity in multiple directions, permitting conformability of the assembly **100** to a variety of membrane surface area configurations in a manner that is substantially independent of the chosen orientation of the assembly **100** during normal use. In various embodiments, a flexural rigidity of at least a portion of the flexible backing **108** is less than a flexural rigidity of at least a portion of the electrode layer **102.**

In general, one advantage of the embodiments of the present invention is realized in minimization of the "footprint" of the assembly **100** when the assembly **100** is applied to a membrane to deliver a composition. As applied herein, the term "footprint" refers to the portion or portions of the assembly **100** that contact a membrane surface area (e.g., a patient's skin) during operation of the assembly **100.** In certain aspects, the surface area of an assembly including the donor electrode **104** and the donor reservoir **134** may be structured to be greater than the surface area of an assembly including the return electrode 106 and the return reservoir **134** to limit the effect of the return assembly on the overall footprint of the assembly **100.** In addition, the length of the distance **152** that provides separation between the anode **104** and cathode **106** may also impact the footprint. Furthermore, the size of the electrodes **104, 106** relative to their respective reservoirs **134, 136** may also affect the footprint of the assembly **100.** In certain aspects, the reservoirs **134, 136** should be at least substantially the same size as their respective electrodes **104, 106.**

It can be appreciated that the inventors have also recognized that once the surface area of the electrode layer **102** is fixed, including configuration of the anode **104** and cathode **106** separation distance **152**, the assembly **100** should be sufficiently flexible and adherent for use on a membrane (e.g., a patient's skin). These objectives may depend on the peripheral area of the transfer adhesive layer **110** that surrounds the stiff electrode layer **102.** In various embodiments, the width of the peripheral area of the transfer adhesive layer **110** adjacent to one or both of the anode **104** and cathode **106** may be provided as a minimum width **137** (as shown, for example, in Figure 4). The minimum width **137** may be structured, in certain aspects, in the range of at least about 0.953 cm (0.375 inches). In turn, these objectives depend on the aggressiveness of the transfer adhesive layer **110** and the flexible backing **108,** which is preferably flexible and compliant as a function of the strength (e.g., modulus of elasticity) and thickness of the flexible backing **108.** Any sufficiently thin material may be flexible (such as ultra-thin PET, for example), but another problem arises in that the transfer adhesive layer **110** and the flexible backing **108** should be capable of removal from a membrane with minimum discomfort to a patient, for example. Consequently, a compliant (i.e., low strength) flexible backing **108** may be employed while maintaining adequate strength for treatments using the assembly **100.**

In various example aspects of the structure of the present invention, the footprint area of the assembly **100** may be preferably in the range of about 3 cm² to 100 cm², more preferably in the range of about 5 cm² to 60 cm², and most preferably in the range of about 20 cm² to 30 cm². In addition, the total electrode **104,106** area may be in the preferred range of about 2 cm² to 50 cm² or more preferably in the range of about 3 cm² to 30 cm², and most preferably in the range of about 4 cm² to 15 cm². The ratio of the area of each reservoir **134, 136** to its corresponding electrode **104, 106** may be, for example, in the range of about 1.0 to 1.5. In one operational example, the total contact area for the electrodes **104, 106** is about 6.3 cm² and the total reservoir **134, 136** contact area is about 7.5 cm². In other aspects, the flexible backing with transfer adhesive 110 for the printed electrode layer **102** may have a thickness in the range of, for example, about 0.038 mm (0.0015 inches) to about 0.013 mm (0.005 inches). The flexible backing 108 may be comprised of a suitable material such as EVA, polyolefins, polyethylene (PE) (preferably low-density polyethylene (LDPE)), polyurethane (PU), and/or other similarly suitable materials.

In other example aspects of the structure of the present invention, the ratio of total electrode surface area to total footprint area may be in the range about 0.1 to 0.7. In certain aspects, the ratio of donor electrode **104** surface area to return electrode **106** surface area may be in the range of about 0.1 to 5.0. In still other aspects, the ratio of donor reservoir **134** thickness to return reservoir **136** thickness may be in the range of about 0.1 to 2.0, or more preferably about 1.0.

In various embodiments, the donor electrode reservoir **134,** for example, may be loaded with an active ingredient from an electrode reservoir loading solution by placing an aliquot of the loading solution directly onto the hydrogel reservoir and permitting the loading solution to absorb and diffuse into the hydrogel over a period of time. Figure 10 illustrates this method for loading of electrode reservoirs in which an aliquot of loading solution is placed on the hydrogel reservoir for absorption and diffusion into the reservoir. Figure 10 is a schematic cross-sectional drawing of an anode electrode assembly **274** including an anode **280** and an anode trace **281** on a backing **288** and an anode reservoir **284** in contact with the anode **280.** An aliquot of a loading solution **285**, containing a composition to be loaded into the reservoir **284** is placed in contact with reservoir **284.** Loading solution **285** is contacted with the reservoir **284** for a time period sufficient to permit a desired amount of the ingredients in loading solution **285** to absorb and diffuse into the gel reservoir **284.** It can be appreciated that any suitable method or apparatus known to those in the art may be employed for loading the reservoir **284** with a composition.

In other embodiments of the present invention, at least one of the hydrogel reservoirs **134, 136** is positioned for electrical communication with at least a portion of at least one of the electrodes **104, 106.** In various aspects, a surface area of at least one of the hydrogel reservoirs **134, 136** may be greater than or equal to a surface area of its corresponding electrode **104, 106.** At least one of the hydrogel reservoirs **134, 136** may be loaded with a composition to provide a loaded hydrogel reservoir below an absorption saturation of the loaded hydrogel reservoir. In addition, at least one component of the assembly **100** in surface contact with, or in the vicinity of, the loaded hydrogel reservoir may have an aqueous absorption capacity less than an aqueous absorption capacity of the loaded hydrogel reservoir. In certain embodiments, a first kind of material comprising the unloaded hydrogel reservoir **134** in electrical communication with the anode electrode **104** is substantially identical to a second kind of material comprising the second unloaded hydrogel reservoir **136** in electrical communication with the cathode electrode **106.**

In other embodiments of the present invention, a slit **202** may be formed in the flexible backing **108** in an area located between the anode **104** and the cathode **106** of the assembly **100.** The slit **202** facilitates conformability of the assembly **100** to a membrane by dividing stress forces between the portion of the assembly including the anode and the portion of the assembly including the cathodes. In various embodiments, the electrode assembly **100** includes one or more non-adhesive tabs **206** and **208** that extend from the flexible backing **108** and to which no type of adhesive is applied. The non-adhesive tabs **206, 208** permit, for example, ready separation of the release cover **138** from its attachment to the electrode assembly **100.** The non-adhesive tabs **206, 208** also may facilitate removal of the assembly **100** from a membrane (e.g., a patient's skin) on which the assembly **100** is positioned for use.

As described above, at least a portion of at least one of the anode electrode trace **112** and the cathode electrode trace **114** may be covered with an insulating dielectric coating **118** at portions along the traces **112, 114.** The insulating dielectric coating **118** may be structured not to extend to cover completely the portion of the traces **112,114** located at the tab end portion **116** of the assembly **100.** This permits electrical contact between the traces **112, 114** and the electrical contacts of an interconnect device such as the flexible circuit connector **250B** of the connector assembly **250.** In various embodiments, the dielectric coating **118** may cover at least a portion of at least one of the anode **104** / reservoir **134** assembly and/or the cathode **106** / reservoir **136** assembly. In addition, the dielectric coating **118** may cover substantially all or at least a portion of a periphery of at least one of the electrodes **104, 106** and/or the traces **112, 114.**

In various embodiments of the present invention, a gap **212** may be provided between a portion of the layer of transfer adhesive **110** nearest to the tab end portion **116** and a portion of the tab stiffener **124** nearest to the layer of transfer adhesive **110** to facilitate removal or attachment of the assembly **100** from/to a component of an electrically assisted delivery device such as the connector assembly **250,** for example. In certain example embodiments, the gap **212** is at least about 1.3 cm (0.5 inches) in width. The gap **212** provides a tactile sensation aid such as for manual insertion, for example, of the assembly 100 into the flexible circuit connector **250B** of the connector assembly **250.** The gap **212** may also provide relief from stress caused by relative movement between the assembly **100** and other components of a delivery device (e.g., the connector assembly **250)** during adhesion and use of the assembly **100** on a membrane.

In addition, at least one tactile feedback notch **214** and one or more wings **216, 218** may be formed in or extend from the tab end **116** of the electrode assembly **100.** The feedback notch **214** and/or the wings **216, 218** may be considered tactile sensation aids that facilitate insertion or removal of the tab end **116** into/from a component of an electrically assisted delivery device such as, for example, to establish an operative association with the flexible circuit connector **250B** of the connector assembly **250.**

Figures 6B and 6C each show the layering of elements of the electrode assembly **100** as shown in Figure 6. In Figures 6B and 6C, it can be seen that the thickness of layers is not to scale and adhesive layers are omitted for purposes of illustration. Figure 6B shows a cross section of the anode electrode **104** / reservoir **134** assembly and the cathode electrode **106** / reservoir **136** assembly. The anode **104** and the cathode **106** are shown layered on the printed electrode layer **102.** The anode reservoir **134** and the cathode reservoir **136** are shown layered on the anode **104** and the cathode **106**, respectively. Figure 6C is a cross-sectional view through the anode **104**, the anode trace **112**, and the anode reservoir **134.** The anode **104**, the anode trace **112** and a sensor trace **130** are layered upon the electrode layer **102.** The anode reservoir **134** is shown in electrical communication with the anode **104**. The tab stiffener **124**, which may be composed of an acrylic material, for example, is shown attached to the tab end **116** of the assembly **100**. In addition, the sensor trace **130** may be located at the tab end **116** of the electrode assembly **100**.

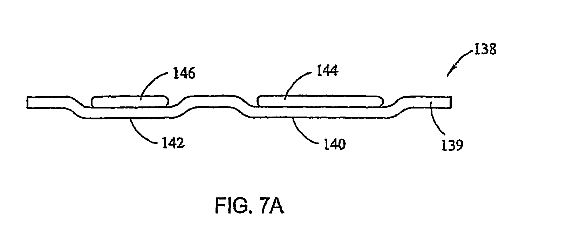

In other embodiments of the present invention, Figures 7 and 7A show schematically the release cover **138** structured for use with various devices, electrode assemblies and/or systems of the present invention. The release cover **138** includes a release cover backing **139**, which includes an anode absorbent well **140** and a cathode absorbent well **142.** In various exemplary aspects, a nonwoven anode absorbent pad may be contained within the anode absorbent well **140** as the transfer absorbent **144**, and a nonwoven cathode absorbent pad may be contained within the cathode absorbent well **142** as the transfer absorbent **146.** In use, the release cover **138** is attached to the electrode assembly **100** so that the anode absorbent pad **144** and the cathode absorbent pad **146** substantially cover the anode reservoir **134** and the cathode reservoir **136,** respectively. The anode absorbent pad **144** and the cathode absorbent pad **146** may each be slightly larger than their corresponding anode reservoir **134** or cathode reservoir **136** to cover and protect the reservoirs **134, 136.** The anode absorbent pad **144** and the cathode absorbent pad **146** may also be slightly smaller than the anode absorbent well **140** and the cathode absorbent well **142,** respectively. In various embodiments, one or more indicia **220** (e.g., a "+" symbol as shown in Figure 2) may be formed on at least a portion of the flexible backing **108** of the assembly **100** adjacent to the anode well **140** and/or the donor well **142.** It can be appreciated that the indicia **220** may promote correct orientation and use of the assembly **100** during performance of an iontophoretic procedure, for example, as well as identifying the drug carrying reservoir-electrode.

The anode absorbent pad **144** and the cathode absorbent pad **146** may be attached to the backing **139** of the release cover **138** by one or more ultrasonic spot welds such as welds **222, 224, 226**, for example, as shown in Figure 7. The welds **222, 224, 226** may be substantially uniformly distributed in areas of connection between the non-woven fabric pads **144, 146** and the wells **140, 142,** respectively.

To facilitate removal of the release cover **138** from the electrode assembly **100,** portions of the backing **139** in communication with the transfer adhesive **110** when the release cover **138** is attached to the electrode assembly **100** may be treated with a release coating, such as a silicone coating, for example.

### Packaging of Iontophorefic Device

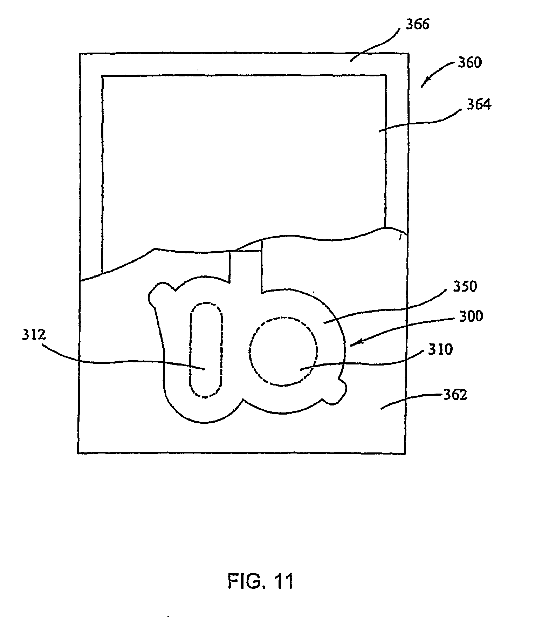

Figure 11 is a breakaway schematic representation of the electrode assembly **300** within a hermetically sealed packaging **360.** Packaged electrode assembly **300** is shown with release liner **350** in place and anode **310** and cathode **312** are shown in phantom for reference. Hermetically sealed packaging **360** is a container that is formed from a first sheet 362 and a second sheet **364,** which are sealed along seam **366.** Hermetically sealed packaging **360** can be of any suitable composition and configuration, so long as, when sealed, substantially prevents permeation of any fluid or gas including, for example, permeation of oxygen into the packaging **360** and/or the loss of water from the packaging **360** after the electrode assembly **300** is sealed inside the hermetically sealed packaging **360**.

In use, sheets **362** and **364** are sealed together to form a pouch after electrode assembly **300** is placed on one of sheets **362** and **364**. Other techniques well-known to those skilled in the art of packaging may be used to form a hermetically sealed package with air or an inert atmosphere. Electrode assembly **300** is then inserted between sheets **362** and **364** and the hermetically sealed packaging **360** is then sealed. The hermetically sealed packaging **360** may be sealed by adhesive, by heat lamination, or by any method known to those skilled in the.art of packaging devices such as electrode-assembly **300**. It should be noted that sheets **362** and **364** may be formed from a single sheet of material that is folded onto itself, with one side of hermetically sealed packaging **360** being a fold in the combined sheet, rather than a seal. In other embodiments, the sheets **362, 364** may be formed from individual sheets that are laminated together, for example, to form a package. Other container configurations would be equally suited for storage of electrode-assembly, so long as the container is hermetically sealed.

Sheets **362** and **364**, and in general, hermetically sealed packaging **360** may be made from a variety of materials. In one embodiment, the materials used to form hermetically sealed packaging **360** has the structure 48 gauge PET (polyethylene terephthalate)/Primer/151b LDPE (low density polyethylene)/0.025 mm (1.0 mil) aluminum foil adhesive/48 gauge PET/10 1b LDPE chevron pouch 0.05 mm (2 mil) peelable layer. Laminates of this type (foil, olefinic films and binding adhesives) form strong and channel-free seals and are essentially pinhole-free, assuring essentially zero transfer of gases and water vapor for storage periods up to and exceeding 24 months. Other suitable barrier materials to limit transport of oxygen, nitrogen and water vapor for periods of greater than 24 months are well-known to those of skill in the art, and include, without limitation, aluminum foil laminations, such as the INTEGRA^{®} products commercially available from Rexam Medical Packaging of Mundelein, Illinois.

It can be appreciated that any of the assemblies, devices, systems, or other apparatuses described herein may be, where structurally suitable, included within hermetically sealed packaging as described above.

### Protocols for Loading Reservoir

In use, electrode reservoirs described herein can be loaded with an active ingredient from an electrode reservoir loading solution according to any protocol suitable for absorbing and diffusing ingredients into a hydrogel. Two protocols for loading a hydrogel include, without limitation, 1) placing the hydrogel in contact with an absorbent pad; a material, such as a nonwoven material, into which a loading solution containing the ingredients is absorbed, and 2) placing an aliquot of the loading solution directly onto the hydrogel and permitting the loading solution to absorb and diffuse into the hydrogel over a period of time.

In applying the first protocol, described above, to the electrode assembly **100**, for example, the loading solutions containing ingredients to be absorbed and diffused into the respective anode reservoir **134** and cathode reservoir **136** are first absorbed into the nonwoven anode absorbent pad **144** and nonwoven cathode absorbent pad **146**, respectively. When a release cover thus loaded is connected to electrode assembly **100**, the ingredients therein desorb and diffuse from the absorbent pads **144** and **146** and into the respective reservoir. In this case, absorption and diffusion from the reservoir cover into the reservoirs has a transfer efficiency of about 95%, requiring that about a 5% excess of loading solution be absorbed into the absorbent pads. Despite this incomplete transfer, the benefits of this loading process, as compared to placing a droplet of loading solution onto the reservoirs and waiting between about 16 and 24 hours or so for the droplet to immobilize and absorb, are great because once the release cover is laminated onto the electrode assembly, the assembly can be moved immediately for further processing and placed in inventory. There is no requirement that the assembly is kept flat and immobile while awaiting completion of absorption and/or diffusion.

The transfer absorbents **144** and **146** are typically a nonwoven material. However, other absorbents may be used, including woven fabrics, such as gauze pads, and absorbent polymeric compositions such as rigid or semi-rigid open-cell foams. In the particular embodiments described herein, the efficiency of transfer of loading solution from the absorbent pads of the release cover to the reservoirs is about 95%. It would be appreciated by those skilled in the art of the present invention that this transfer efficiency will vary depending on the composition of the absorbent pads and the reservoirs as well as additional physical factors including, without limitation, the size, shape, and thickness of the reservoirs and absorbent pads and the degree of compression of the absorbent pad and reservoir when the release cover is affixed to the electrode assembly. The transfer efficiency for any given release cover-electrode assembly combination can be readily determined empirically and, therefore, the amount of loading solution needed to fully load the reservoirs to their desired drug content can be readily determined to target specifications.

As discussed above, Figure 10 illustrates the second protocol for loading of electrode reservoirs in which an aliquot of loading solution is placed on the hydrogel reservoir for absorption and diffusion into the reservoir. The transfer absorbents **144, 146** typically are not included in the release cover for electrode assemblies having reservoirs loaded by this method.

In various embodiments, the electrode assembly **100** is manufactured, in pertinent part, by the following steps. First, electrodes **104** and **106** and traces **112, 114** and **130** are printed onto a polymeric backing, such as treated ink-printable treated PET film, for example, or another suitably semi-rigid dimensionally stable material. The dielectric layer **118** may then be deposited onto the appropriate portions of traces **112** and **114** that are not intended to electrically contact the electrode reservoirs and contacts of an interconnect between the electrode assembly and a power supply/controller, for example. The polymeric backing onto which the electrodes are printed is then laminated to the flexible backing **108.** The anode reservoir **134** and cathode reservoir **136** are then positioned onto the electrodes **104** and **106,** respectively. In the assembly of the release cover **138** for devices loaded under the first protocol, the transfer absorbents **144** and **146** are ultrasonically spot welded within wells **140** and **142** and are loaded with an appropriate loading solution for absorption and/or diffusion into the anode and/or cathode reservoirs **134** and **136.** An excess of about 5% loading solution (over the amount needed to absorb and diffuse into the hydrogel) typically is added to the reservoir covers due to the about 95% transfer efficiency of the loading process, resulting in some of the loading solution remaining in the absorbent reservoir covers.

### Methods of Use

Once assembled and loaded with loading solution under the first protocol, the release cover is positioned on the electrode assembly **100** with the loaded transfer absorbents **144** and **146** in surface contact with anode and cathode reservoirs **134** and **136,** respectively. Over a time period, typically at least about 24 hours, substantial portions (about 95%) of the loading solutions are absorbed and diffused into the hydrogel reservoirs. The completed assembly is then packaged in an optional inert gas environment and hermetically sealed.

In one method of use, the release cover **138** is removed from the electrode assembly **100**, and the electrode assembly **100** is placed on a patient's skin at a suitable location. After the electrode assembly **100** is placed on the skin, it is inserted into a suitable interconnect, such as a component of the connector assembly **250,** for example. An electric potential is applied according to any profile and by any means for electrically assisted drug delivery known in the art. Examples of power supplies and controllers for electrically assisted drug delivery are well known in the art, such as those described in U.S. Patent Nos. 6,018,680 and 5,857,994, among others. Ultimately, the optimal current density, drug concentration and current profile (with time) and/or electric potential is determined and/or verified experimentally for any given electrode/electrode reservoir combination.

The electrodes described herein are standard Ag or Ag/AgCl electrodes and can be prepared in any manner according to standard methods in such a ratio of Ag to AgCl (if initially present), thickness and pattern, such that each electrode will support the Ag/AgCl electrochemistry for the desired duration of treatment. Typically, as is common in preparation of disposable iontophoresis electrodes, the electrodes and electrode traces are prepared by printing Ag/AgCl ink in a desired pattern on a stiff polymeric backing, for example 0.051 mm (0.002 inch) print treated PET film, by standard printing methods.. Ag/AgCl ink is commercially available from E.I. du Pont de Nemours and Company, for example and without limitation, du Pont Product ID Number 5279. The dielectric also may be applied to the electrode traces by standard methods. As with the electrode, dielectric ink may be applied in a desired pattern over the electrodes and electrode traces by standard printing methods, for example, by rotogravure or screen printing.

The pressure-sensitive adhesive (PSA) and transfer adhesives may be any pharmaceutically acceptable adhesive suitable for the desired purpose. In the case of the pressure-sensitive adhesive, the adhesive may be any acceptable adhesive useful for affixing an electrode assembly to a patient's skin or other membrane. For example, the adhesive may be polyisobutylene (PIB) adhesive. The transfer adhesive, used to attach different layers of the electrode assembly to one another, also may be any pharmaceutically acceptable adhesive suitable for that purpose, such as PIB adhesive. For assembly of the electrodes described herein, the PSA typically is provided pre-coated on the backing material with a silicone-coated release liner attached thereto to facilitate cutting and handling of the material. Transfer adhesive typically is provided between two layers of silicone-coated release liner to facilitate precise cutting, handling and alignment on the electrode assembly.

The anode and cathode reservoirs described herein may comprise a hydrogel. The hydrogel typically is hydrophilic and may have varying degrees of cross-linking and water content, as is practicable. A hydrogel as described herein may be any pharmaceutically and cosmetically acceptable absorbent material into which a loading solution and ingredients therein can be absorbed, diffused, or otherwise incorporated and that is suitable for electrically assisted drug delivery. Suitable polymeric compositions useful in forming the hydrogel are known in the art and include, without limitation, polyvinylpyrrolidone (PVP), polyethyleneoxide, polyacrylamide, polyacrylonitrile and polyvinyl alcohols. In one non-limiting embodiment, the hydrogel may comprise about 15% to about 17% PVP by weight. The reservoirs may optionally contain additional materials such as, without limitation: preservatives, such as Phenonip Antimicrobial, available commercially from Clariant Corporation of Mount Holly, North Carolina; antioxidants, such as sodium metabisulfite; chelating agents, such as ethylenediamine tetraacetic acid (EDTA); and humectants may also be incorporated. A typical unloaded reservoir contains preservatives and salt. As used herein in reference to the water component of the electrode reservoirs, the water is purified and preferably meets the standard for purified water in the USP XXIV.

As discussed above, the hydrogel has sufficient internal strength and cohesive structure to substantially hold its shape during processing, forming, and during its intended use and leave essentially no residue when the electrode is removed after use. As such, the cohesive strength of the hydrogel and the adhesive strength between the hydrogel and the electrode are each greater than the adhesive strength of the bonding between the hydrogel and the membrane (for instance skin) to which the electrode assembly is affixed in use. In one non-limiting embodiment, the hydrogel may have a thickness from about 0.089 cm (0.035 in.) to about 0.114 cm (0.045 in.). In another embodiment, the hydrogel may have a thickness from about 0.013 cm (0.005 in.) to about 0.38 cm (0.015 in). In still another embodiment, the hydrogel may have a thickness from about 0.38 cm (0.015 in.) to about 0.064 cm (0.025 in). Hydrogel thicknesses set forth herein are measured prior to loading the hydrogel with the loading solution.

The unloaded donor (anode) reservoir also includes a salt, preferably a fully ionized salt, for instance a halide salt such as sodium chloride, in a concentration of from about 0.001 wt. % to about 1.0 wt. %, preferably from about 0.01 wt. % to about 0.09 wt. %, and most preferably about 0.06 wt. %. The salt content is sufficient to prevent electrode corrosion during manufacture and shelf-storage of the electrode assembly. These amounts may vary for other salts in a substantially proportional manner depending on a number of factors, including the molecular weight and valence of the ionic constituents of each given salt in relation to the molecular weight and valence of sodium chloride. Other salts, such as organic salts, are useful in ameliorating the corrosive effects of certain drug salts. Typically, the best salt for any ionic drug will contain an ion that is the same as the counter ion of the drug. For instance, acetates would be preferred when the drug is an acetate form, such as GnRH acetate. However, the aim of the salt is to prevent corrosion of the electrodes.

In one embodiment, GnRH is used to elicit a desired pharmacological response. If the counterion of GnRH is not chloride, for example acetate in GnRH acetate, though chloride ions may be useful to prevent electrode corrosion, a corrosion-inhibiting amount of that other counterion may be present in the unloaded reservoir in addition to, or in lieu of, the chloride ions to prevent corrosion of the electrode. If more than one counterion is present, such as in the case where more than one drug is loaded and each drug has a different counterion, it may be preferable to include sufficient amounts of both counterions in the reservoir to prevent electrode corrosion.

In certain embodiments, a composition comprising GnRH is added in the drug loading solution. GnRH in the loading solution can comprise any amount necessary to elicit the desired pharmacological response. GnRH loading concentrations in the loading solutions for use in the various non-limiting embodiments can be from about 0.1 mg/mL to about 150 mg/mL. In certain embodiments, the GnRH concentration in the loading solution can be from about 5 mg/mL to about 75 mg/mL. In other embodiments, the GnRH concentration in the loading solutions can be from about 10 mg/mL to about 100 mg/mL. In certain non-limiting embodiments, the GnRH in the loading solution has a concentration of about 60 mg/mL. In other non-limiting embodiments, the GnRH in the loading solution has a concentration of about 20 mg/mL. In other non-limiting embodiments, the GnRH in the loading solution has a concentration of about 25 mg/mL. In still other non-limiting embodiments, the GnRH in the loading solution has a concentration of about 50 mg/mL.

For example, for an iontophoretic device having an anode reservoir having a volume of about 0.705 mL, according to one embodiment of the present disclosure, the GnRH loading weight, according to certain embodiments, may be from about 0.07 mg GnRH to about 106 mg GnRH. In certain embodiments, the GnRH weight in the anode reservoir can be from about 3.5 mg GnRH to about 53 mg GnRH. In other embodiments, the GnRH weight in the anode reservoir can be from about 7 mg GnRH to about 70 mg GnRH. In certain non-limiting embodiments, the GnRH in the anode reservoir has a weight of about 42 mg. In other non-limiting embodiments, the GnRH in the anode reservoir has a weight of about 14 mg. In other non-limiting embodiments, the GnRH in the anode reservoir has a weight of about 18 mg. In still other non-limiting embodiments, the GnRH in the anode reservoir has a weight of about 35 mg. One having ordinary skill in the art would understand that the weight of the GnRH in the anode reservoir may be dependent on, for example, the reservoir volume, molecular weight of the GnRH formulation used (i.e., hydrochloride salt, acetate salt, free base, etc.) and/or concentration of loading solution, and would be able to calculate the desired amount of GnRH, given the reservoir volume and loading solution concentration.

In another embodiment, a composition comprising one or more analog of GnRH is added in the drug loading solution. Suitable GnRH analogs include, but are not limited to, Buserelin, Deslorelin, Goserelin, Histrelin, Leuprolide (Leuprorelin), Nafarelin, and Triptorelin. One potential advantage of utilizing a GnRH analog, in lieu of or in addition to GnRH, is that lower concentrations of the active ingredient may be loaded into the device and thereafter administered to the patient. For example, loading concentrations of GnRH analogs in the loading solutions can be from about 0.1 mg/mL to about 50 mg/mL. Thus, for a reservoir volume, for example, of 0.705 mL, according to certain embodiments, the GnRH analogs may have a loading weight of from about 0.07 mg to about 35 mg. A second advantage of utilizing a GnRH analog is that said analog may have a longer half-life than GnRH.

The loading solution according to various non-limiting embodiments disclosed herein, may be formed by dissolving GnRH, or a salt thereof, for example an HCl salt or an acetate salt, in water, wherein the pH of the water is in the range from about 4 to about 8. In certain embodiments, the pH of the water is about 5.0. In other non-limiting embodiments, the loading solution comprising one or more analogs of GnRH, as discussed above, or a pharmaceutically acceptable salt thereof, is dissolved in water having a pH from about 4 to about 8. Other components, such as, but not limited to, one or more electrolyte, such as salt (NaCl), disodium EDTA, citric acid, sodium metabisulfite, and glycerin may be dissolved in the water, either before the addition of the GnRH and/or GnRH analog, concomitant with the addition of the GnRH and/or GnRH analog, or after the addition of the GnRH and/or GnRH analog.

The return (cathode) reservoir may be a hydrogel with the same or different polymeric structure and typically contains a salt such as sodium chloride, a preservative and, optionally, a humectant. Depending upon the ultimate manufacturing process, certain ingredients may be added during cross-linking of the hydrogel reservoir, while others may be loaded with the active ingredients. Nevertheless, it should be recognized that irrespective of the sequence of addition of ingredients, the salt must be present in the reservoir adhering to the electrode and substantially evenly distributed therethrough prior to the loading of the active ingredient(s) or other ingredient(s) to limit the formation of electrolytic concentration cells.

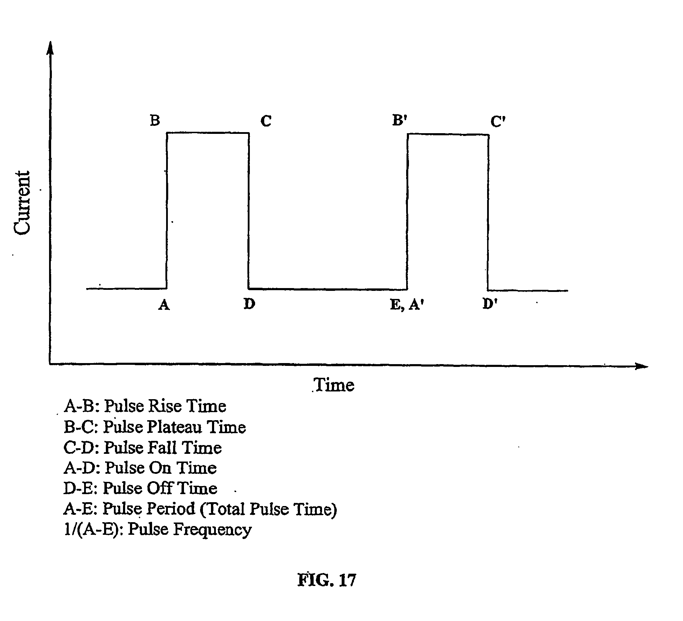

The iontophoretic delivery system, according to the various non-limiting embodiments disclosed herein, is capable of delivering a wide variety of current profiles. According to certain non-limiting embodiments, the current profiles are of a periodic nature, i.e., a certain profile is repeated periodically, for example, comprising a plurality of individual pulses. In addition, according to certain non-limiting embodiments, the current profiles are of a unipolar or unidirectional nature, i.e., the current levels are all positive or all negative. One non-limiting example of a current profile is represented in Figure 17, which shows a schematic of rectangular periodic current profile (A-B-C-D-E). Referring now to Figure 17, the profile region A-B represents the rise time of the pulse, profile region B-C represents the pulse plateau, profile region C-D represents the fall (or decline) time of the pulse, and profile region D-E represents the off-time of the pulse. The total on-time of the pulse is represented by the region A-D, and the pulse duty cycle is defined as the percent or ratio of the pulse on-time (A-D) to the total pulse time A-E, including both the pulse on-time and pulse off-time (i.e., [(A-D)/(A-E)] x 100%). The pulse period is defined as the total pulse time (A-E) and the pulse frequency (Hz) is the inverse of the pulse period (i.e., pulse frequency = 1/(A-E)). In certain non-limiting embodiments, the first pulse profile is followed by at least one subsequent pulse profile, represented in Figure 17 by rectangular pulse profile (A'-B'-C'-D'-E'). In certain non-limiting embodiments, the at least one subsequent pulse profile has the same magnitude as rectangular pulse profile A-B-C-D-E. In other non-limiting embodiments, the at least one subsequent pulse profile has a different magnitude as pulse profile A-B-C-D-E, for example, in certain non-limiting embodiments, the current of the at lease one subsequent pulse profile is less that the current of pulse profile A-B-C-D-E. Whereas only rectangular periodic current profiles have been described herein for the purpose of illustrating one embodiment disclosed herein and not for the purpose of limiting the same, it will be appreciated by those having skill in the art that numerous variations of the current profile (rectangular, square, triangular, sawtooth, sinusoidal, and combinations thereof as needed) may be used within the principle and scope of the invention without departing from the invention.

According to certain non-limiting embodiments, the current delivery profile comprises n pulses, where n is greater than or equal to 2, the pulse current profile is unidirectional, and substantially rectangular in shape. According to certain non-limiting embodiments, the pulse frequency (cycles per second, Hz) ranges from about 1.0 x 10⁻⁴ Hz to about 5 x 10⁻⁴ Hz. According to certain non-limiting embodiments, the on-time may range from about 1 minute to about 15 minutes, more preferably from about 5 minutes to about 10 minutes. According to certain non-limiting embodiments, the pulse duty cycle ranges from about 1% to about 20%, more preferably from about 5% to about 10%, and most preferably about 5.5% and about 8.9%.

In certain non-limiting embodiments, the current delivery profile may comprise from 4 to 10 pulses having a duration from 4 to 10 minutes each and a current of from about 1 mA to about 4 mA with a rest time (where current = 0 mA) between pulses of from 60 minutes to 110 minutes. In another non-limiting embodiment, the current delivery profile may comprise 5 pulses, having an 8 minute duration each and a current of 1.2 mA with a rest time between pulses of from 80 minutes to 90 minutes. In another non-limiting embodiment, the current delivery profile may comprise 8 pulses having a 5 minute duration each and a current of 1.2 mA with a rest time between pulses of from 80 minutes to 90 minutes.

### Patch Fabrication Platform I - Pre-loaded Integrated Patch

In one non-limiting embodiment, the following components were assembled to prepare an electrode assembly, essentially as shown in figures 2 through 9, and 11, as discussed above, for delivery of GnRH by iontophoresis. The patch system used in the study described above was an integrated patch comprising an active electrode (anode) and a return electrode (cathode). Each patch was constructed of an Ag/AgCl electrode laminate, a polyvinylpyrrolidone (PVP) hydrogel reservoir, a backing film/acrylic adhesive laminate, and a siliconized release liner. The electrode material used for the construction of the anode and cathode was a Ag/AgCl printed ink material on a polyester substrate and did not exceed 5 cm² in area. The patch fabrication is discussed in greater detail as follows.

**Backing:** ethylene vinyl acetate (EVA) (0.10 mm (4.0 mil) ± 0.01 mm (0.4 mil)) coated with polyisobutylene (PIB) adhesive (6 mg/cm²) (Adhesive Research of Glen Rock, Pennsylvania). The backing was dimensioned to yield a gap of between 0.939 cm (0.370 inches) and 0.953 cm (0.375 inches) ± 0.013 cm (0.005 inches) between the gel electrode and the outer edge of the backing at any given point on the edge of the gel. Excluding the tactile feedback notch and the wings, the tab end of the electrode had a width of 1.14 cm (0.450 inches) to 1.27 cm (0.500 inches) ± 0.013 cm (0.005 inches).

**Tab stiffener:** 0.18 mm (7 mil) PET/acrylic adhesive (Scapa Tapes of Windsor, Connecticut).

Printed electrode: Ag/AgCl electrode printed on du Pont 200 J102 0.05 mm (2 mil) clear printable PET film with dielectric coated Ag/AgCl traces. The Ag/AgCl ink was prepared from du Pont Ag/AgCl Ink #5279, du Pont Thinner #8243, du Pont Defoamer and methyl amyl ketone (MAK). The dielectric ink was Sun Chemical Dielectric Ink # ESG56520G/S. The electrodes were printed substantially as shown in Figures 2 and 4, with a coatweight of both the electrode ink and the dielectric ink of at least about 2.6 mg/cm². The anode had a diameter of 2.26 cm (0.888 inches) ± 0.013 cm (0.005 inches). The cathode was essentially oval shaped, as shown in the figures. The semicircular ends of the oval both had a radius of 0.490 cm (0.193 inches) ± 0.013 cm (0.005 inches). The centers of the semicircular ends of the oval were separated by 1.84 cm (0.725 inches) ± 0.013 cm (0.005 inches).

**Transfer Adhesive:** 6 mg/cm² ± 0.4 mg/cm², Ma-24A PIB transfer adhesive, (Adhesives Research). When printed onto the electrode, there was a gap of 0.076 cm (0.030 inches) ± 0.0076 cm (0.0030 inches) between the anode and cathode electrodes and the transfer adhesive surrounding the electrodes.

**Anode Gel Reservoir:** 1.0 mm (40 mil) high adhesion crosslinked polyvinylpyrrolidone (PVP) hydrogel sheet containing: 24% wt. ± 1% wt. PVP; 1% wt. ± 0.05% wt. Phenonip; 0.06% wt. NaCl to volume (QS) with purified water (USP).

The hydrogel was crosslinked by electron beam irradiation at an irradiation dose of about 2.7 Mrad (27 kGy) at an electron beam voltage of 1 MeV. The anode gel reservoir was circular, having a diameter of 2.525 cm (0.994 inches) ± 0.013 cm (0.005 inches) and has a mass of about 0.53 g. The reservoir was loaded by placing 334 mg of Loading Solution (see Table A) onto the absorbent (non-woven), described below, and then placing the cover assembly containing the absorbent onto the patch so that the absorbent contacts the anode reservoir directly, permitting the loading solution to absorb into the reservoir.

Loading Solution Compositions were prepared from the ingredients shown in Table A, resulting in Anode Reservoir Composition as presented in Table B. For a 10 mg/mL loaded patch, a loading concentration of 25.9 mg/mL was used. For a 25 mg/mL loaded patch, a loading concentration of 64.8 mg/mL was used. For a 50 mg/mL loaded patch, a loading concentration of 129.6 mg/mL was used.

**Table A: Loading Solution Compositions**

| Ingredient | 2 mg/mL Loaded patch | 10 mg/mL Loaded patch | 25 mg/mL Loaded patch | 50 mg/mL Loaded patch |
|---|---|---|---|---|
| GnRH (HCl)* | 5.18 mg/mL | 25.9 mg/mL | 64.8 mg/mL | 129.6 mg/mL |
| NaCl | 9.0 mg/mL | 9.0 mg/mL | 9.0 mg/mL | 9.0 mg/mL |
| NaOH | 3.7 mg/mL | 3.7 mg/mL | 3.7 mg/mL | 3.7 mg/mL |
| USP Water | 982.1 mg/mL | 961.4 mg/mL | 922.5 mg/mL | 857.6 mg/mL |

| | | | | |
|---|---|---|---|---|
| *Assay 0.87 GnRH | | | | |

**Table B: Composition of All Components in Fully Loaded Hydrogel Anode**

| Ingredient | 10 mg/mL Loaded patch | 25 mg/mL Loaded patch | 50 mg/mL Loaded patch |
|---|---|---|---|
| GnRH (HCl) | 0.8% | 2.1% | 4.2% |
| NaCl | 0.4% | 0.4% | 0.4% |
| NaOH | 0.1% | 0.1% | 0.1% |
| Water | 82.8% | 81.4% | 79.0% |
| PVP | 15.1% | 15.1% | 15.1% |
| Phenoxyethanol+parabens | 0.6% | 0.6% | 0.6% |

**Cathode Reservoir:** The unloaded cathode gel consisted of a 1.0 mm (40 mil) high adhesion polyvinylpyrrolidone (PVP) hydrogel sheet containing: 24% ± 1 % wt. PVP, 1% Phenonip antimicrobial, 0.06 % wt. NaCl and purified water (Hydrogel Design Systems, Inc.). The hydrogel was crosslinked by electron beam irradiation at an irradiation dose of about 2.7 Mrad (27 kGy) at an electron beam voltage of 1 MeV. The cathode reservoir was essentially oval shaped, as shown in the figures The semicircular ends of the oval both had a radius of 0.617 cm (0.243 inches) ± 0.013 cm (0.005 inches). The centers of the semicircular ends of the oval were separated by 1.85 cm (0.725 inches) ± 0.013 cm (0.005 inches) and the volume of the cathode reservoir was about 0.36 mL (0.37 g). The cathode reservoir was loaded by placing 227 mg of cathode loading solution, described below onto the absorbent (non-woven) described below and then placing the cover assembly containing the absorbent onto the patch so that the absorbent contacts the cathode reservoir directly, permitting the loading solution to absorb into the reservoir. Cathode loading Solution was prepared from the ingredients shown in Table C, resulting in a cathode reservoir composition as presented in Table D.

**Table C: Cathode Loading Solution**

| **Ingredient** | **% Wt.** |
|---|---|
| Glycerin | 30 |
| NaCl | 1.28 |
| Phenoxyethanol-parabens mixture | 0.10 |
| Sodium Phosphate monobasic | 6.23 |
| Water | QS |

**able D: Cathode Reservoir Composition**

| **INGREDIENT** | **mg/Patch** | **FUNCTION** |
|---|---|---|
| Glycerin | 68.3 | Humectant |
| Sodium Chloride | 3 | Anti-corrosion Agent |
| Monobasic Sodium Phosphate | 14.2 | Acidulating Agent |
| Phenoxy ethanol + Parabens | 3.3 | Preservative |
| PVP | 89 | Physical Structure |
| Water | 419 | Vehicle, Mobile Phase |

Within-lot variation in solution doses and composition typically is ±5%, but has not been analyzed statistically.

**Release cover:** 0.19 mm (7.5 mil) ± 0.0095 mm (0.375 mil) polyethylene terephthalate glycolate (PETG) film with silicone coating (Furon 7600 UV-curable silicon).

**Nonwoven:** 1.00 mm ± 0.2 mm Vilmed M1561 Medical Nonwoven, a blend of viscose rayon and polyester/polyethylene (PES/PE) fibers thermal-bonded to PE (Freudenberg Faservliesstoffe KG Medical Nonwoven Group of Weinheim, Germany).

**Electrode Assembly:** The electrode was assembled substantially as shown in the figures, with the anode and cathode reservoirs laminated to the electrodes. The tab stiffener was attached to the tab end of the backing of electrode assembly on the opposite side of the backing from the anode and cathode traces. The drugs were added to the unloaded anode reservoir as indicated above.

**Packaging:** The assembled electrode assembly was hermetically sealed in a foillined polyethylene pouch.

### Patch Fabrication Platform II - Drop Loaded Integrated Patch

In another embodiment, an electrode assembly was prepared with all of the features that are in Figures 2-11, as described herein, except that there is no absorbent, as set forth in Figures 3 and 7A. According to this embodiment, unloaded gel reservoirs within an integrated patch assembly were prepared as follows to the specifications shown in Table E:

**Table E: Unloaded Gel Reservoir Content**

| **Ingredient** | **% Wt.** |
|---|---|
| PVP | 24.0 |
| Phenonip antimicrobial (phenoxy ethanol and parabens) | 1.0 |
| NaCl | 0.06 |
| Purified water | QS |

The gels were crosslinked by electron beam irradiation at an irradiation dose of about 2.7 Mrad (27 kGy) at an electron beam voltage of 1 MeV.

In the present embodiment, the gel reservoirs were loaded by the droplet loading method, as described herein. The unloaded anode gel reservoirs were placed on Ag/AgCl anodes and 0.32 mL aliquots of Loading Solution (as shown in Table A) were placed on the reservoirs and were permitted to absorb and diffuse into the reservoir.

### Example 1

This Example assessed the feasibility and reproducibility of GnRH delivery by transdermal iontophoresis using the device of Patch Fabrication Platform I relative to subcutaneous drug delivery. Using 10 mg/mL GnRH concentration, 0.24 mA/cm² current density, 5 cm² active area, and 8 min pulse application, iontophoresis produced plasma profiles comparable to subcutaneous injection over 5 dosing cycles. The dose delivered and reproducibility of drug delivery were close to or within acceptable criteria.

Six prepubescent female Yorkshire pigs were used for this Example. A crossover design was used whereby each animal received both subcutaneous (SC) and iontophoretic treatments. Subcutaneous and iontophoresis experiments were spaced two weeks apart to meet acceptable animal blood draw limits. The subcutaneous injection experiments were performed first in the sequence. During the two week study the animal weights ranged from 15-32 kg.

The animal hair were clipped the night before the experiment. Prior to applying patches, a skin site free of obvious cuts and scrapes was selected. The selected skin site was wiped clean with warm water and an alcohol-loaded gauze pad and patted dry.

The subcutaneous dosing solutions of concentration 200 µg/mL and 400 µg/mL were prepared in pH 5.0 water (pH of maximum stability). Pigs 1 and 2 received 50 µl of 400 µg/mL solution, while pigs 3-6 received 100 µl of 200 µg/mL solution for each of the 5 doses.

Iontophoretic hydrogel patches prepared according to Patch Fabrication Platform I (5 cm² active area) were used in these studies. The patches were loaded with GnRH (HCl) solution to provide final concentration of either 10 mg/mL GnRH (HCl) or 2 mg/mL GnRH (HCl) in the patch. The pH of the gel surface was from about 5 to about 5.5.

All animals were placed in a sling under general propofol anesthesia and jugular, ear vein and arterial catheters placed percutaneously. For subcutaneous injection studies, a venous catheter was placed against the lower abdominal wall of the animal and 20 µg of drug was injected at 90-min intervals (total 5 dosing cycles). Each drug injection was followed by saline flush. The subcutaneous injection study in each animal was followed by iontophoresis administration in the same animal two weeks later. The iontophoresis experiments also involved five dosing cycles at 90-minute intervals. The drug loaded patches were placed on the back of the animal just off the midline section.

A laboratory controller was used as a constant current source. The controller is a battery operated electrotransport controller and delivers a preprogrammed current density profile for experimental studies. In addition, the controller measures the voltage across and the current through an electrotransport patch during drug delivery. The accuracy of the delivered current is ± 1% of full-scale. The controller has a maximum voltage of 35 V and can take readings once per second for up to an hour. The accuracy of the recorded current is ± 1% of full-scale. The accuracy of the recorded voltage is 0.8 V Max. There are no moving parts on the controller. Infrared transmission of the delivery profile and data retrieval to a computer ensures there are no disturbances during the treatment of a subject.

The iontophoretic conditions, each using 3 pigs, are summarized in Table F, below. Example 1 utilized a current delivery profile comprising five cycles of eight minute pulses each having a current of 1.2 mA, with rest times of 90 minutes between pulses.

**Table F: Experimental Conditions (Example 1)**

| *Drug Concentration in patch* | *Current* | *Time* |
|---|---|---|
| 10 mg/mL GnRH (Condition #1) | 1.2 mA | 8 minute pulse |
| 2 mg/mL GnRH (Condition #2) | 1.2 mA. | 8 minute pulse |

Blood samples were drawn from the jugular catheter into pre-chilled 3 mL vacutainer tubes using the following protocol. The tubes contained EDTA (1 mg/mL of blood) and Aprotinin (500 KIU/mL of blood). A 2 mL flush volume of blood was drawn followed by a 3 mL sample. The blood samples were withdrawn as outlined in Table G below. The tubes were centrifuged within 15 min of blood collection at 1600 rpm for 15 min at 4°C. The separated plasma was split into two (approximately equal volume) 2 mL tubes and frozen on dry ice. Samples were stored at -70°C until shipment. The samples were analyzed using an I¹²⁵ radio labeled immunoassay.

**Table G: Blood Sample Times (Example 1)**

| Time (min) | Pulse 1 | Pulse 2 | Pulse 3 | Pulse 4 | Pulse 5 |
|---|---|---|---|---|---|
| -15 | X | | | | |
| 0 | X | X | X | X | X |
| 2 | X | X | X | X | X |
| 5 | X | X | X | X | X |
| 10 | X | X | X | X | X |
| 15 | X | X | X | X | X |
| 20 | X | X | X | X | X |
| 30 | X | X | X | X | X |
| 45 | X | X | X | X | X |
| 60 | X | X | X | X | X |
| 90 (85 for ionto) | | | | | X |
| 105 | | | | | X |
| 24 hr | | | | | X |

| | | | | | |
|---|---|---|---|---|---|
| X = blood sample taken. Total 49 blood samples at 3.0 mL =147 mL | | | | | |

All experimental animals were allowed to recover from anesthesia after subcutaneous treatment. The jugular catheter was left over night and removed after 24 hour blood draw was complete. Immediately following the 24 hour iontophoresis blood draw, animals were euthanized. Animal 1 died seven days after subcutaneous treatment. Animals 2, 3, and 4 received iontophoresis under condition #1 and animals 5 and 6 received iontophoresis under condition #2.

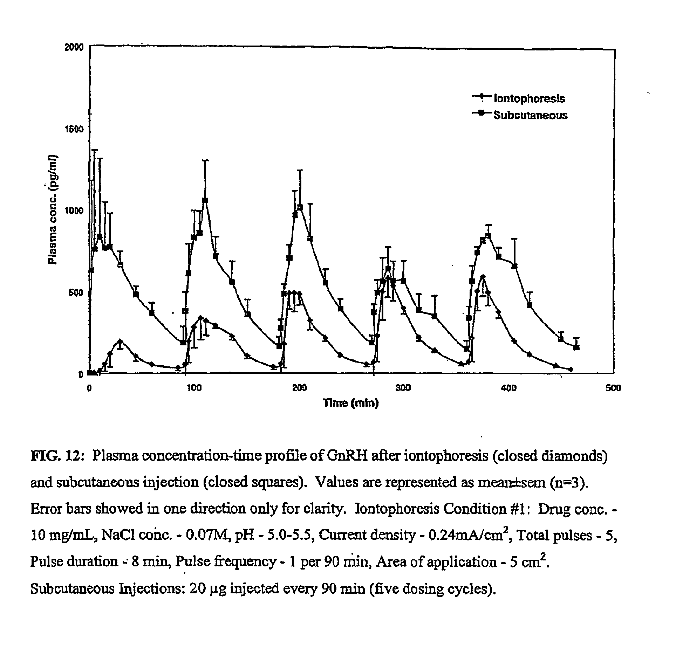

Fig. 12 shows mean plasma concentration-time profile after subcutaneous injection and iontophoresis condition #1 (n=3). The general shape of plasma concentration-time profiles following iontophoresis are very similar to subcutaneous injection profile. Table H shows pharmacokinetic ("PK") parameters (area under the curve ("AUC"), maximum concentration of drug in the bloodstream in a set period of time ("Cₘₐₓ"), and time at which the drug is at the maximum concentration in the bloodstream ("Tₘₐₓ") obtained after iontophoresis condition #1 and subcutaneous injection. Mean Tₘₐₓ after iontophoresis condition #1 and subcutaneous injection was comparable. The percent difference between iontophoresis and subcutaneous means was 47% of the subcutaneous AUC (20 µg dose). The mean AUC for pulse 1 was lower than the next four pulses. Mean coefficient of variation ("CV") (Standard Deviation/mean x 100) (for AUC) for iontophoretic drug delivery was 35% as compared to 18% for subcutaneous drug delivery. If pulse 1 is excluded, mean CV (for AUC) for iontophoretic drug delivery becomes 15% which is better than CV for subcutaneous drug delivery of 18%. The percent difference between iontophoresis and subcutaneous Cₘₐₓ means was 32% of the subcutaneous mean Cₘₐₓ. Mean CV (for Cₘₐₓ) for iontophoretic drug delivery was 40% as compared to 20% for subcutaneous drug delivery. If pulse 1 is excluded, mean CV (for Cₘₐₓ) for iontophoretic drug delivery is 22% which is comparable to CV for subcutaneous drug delivery of 20%. The ratio of the mean CV for Cₘₐₓ between iontophoretic drug delivery and subcutaneous drug delivery was 2.0 and for AUC the ratio was 1.9. This ratio for Cₘₐₓ improved to 1.1 and for AUC to 0.83, if pulse 1 was excluded. The data discussed above is animal weight normalized. These results show that iontophoretic delivery of GnRH is feasible and reproducible relative to subcutaneous drug delivery.

**Table H: PK parameters (AUC, Cₘₐₓ and Tₘₐₓ) after subcutaneous and iontophoretic delivery of GnRH (condition #1, n=3, animals 2-4)**

| **Parameter** | **Pulse 1** | **Pulse 2** | **Pulse 3** | **Pulse 4** | **Pulse 5** | **Mean±SD** | **CV** |
|---|---|---|---|---|---|---|---|
| Ionto. AUC (pg-min/mL) | 6941±5158 | 15508±3285 | 20343±8289 | 17367±7324 | 20474±10571 | 16127±5545 | 34% |
| | 9488±6645* | 21261±10198 | 28178±10949 | 25513±11747 | 30137±16667 | 22915±8210 | 35% |
| Subcut. AUC (pg-min/mL) | 44519±22292 | 48560±17637 | 49860±15085 | 30855±9268 | 50532±10389 | 44865±8172 | 18% |
| Ionto. Cₘₐₓ (pg/mL) | 192±140 | 364±206 | 535±197 | 574±370 | 565±290 | 446±165 | 37% |
| | 262±177* | 495±255 | 743±272 | 847±575 | 831±458 | 636±252 | 40% |
| Subcut. Cₘₐₓ (pg/mL) | 1074±778 | 1064±423 | 1036±368 | 639±229 | 856±114 | 934±187 | 20% |
| Ionto. Tₘₐₓ (min.) | 30±0 | 22±8 | 15±5 | 18±4 | 15±0 | 20±6 | 30% |
| Subcut. Tₘₐₓ (min) | 22±14 | 17±6 | 17±3 | 15±0 | 25±17 | 19±4 | 21% |

| | | | | | | | |
|---|---|---|---|---|---|---|---|
| "Ionto" = iontophoresis, "Subcut" = subcutaneous * the second row for Ionto., AUC and Cₘₐₓ is normalized by animal weight | | | | | | | |

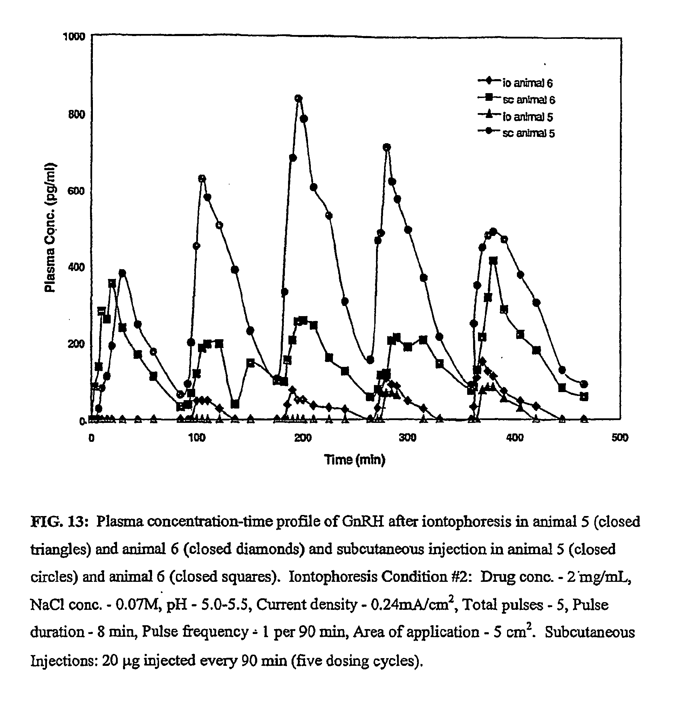

Fig. 13 shows plasma concentration-time profile after subcutaneous injection and iontophoresis condition #2. For animal 5, plasma concentrations for the first 3 pulses were below assay limit of detection and for animal 6, plasma concentrations for the first pulse was below assay limit of detection. For animal 5, pulses 4 and 5 and pulses 2-5 for animal 6 again followed the same general profile as subcutaneous injection and iontophoresis condition #1 but the delivery was considerably lower. Table I and J show PK parameters (AUC, Cₘₐₓ and Tₘₐₓ) obtained after iontophoresis condition #2 and subcutaneous injection in animals 5 and 6, respectively. Mean AUC and Cₘₐₓ for pulses 4 and 5 (4103 pg-min/mL and 138 pg/mL) were 5.6 fold and 4.6 fold less than iontophoresis condition #1, respectively. This is in agreement with the difference in GnRH concentration in drug patches used for two conditions (10 mg/mL for iontophoresis condition #1 and 2 mg/mL for iontophoresis condition #2, a 5 fold difference). The Cₘₐₓ and AUC showed a general increasing trend from pulse 1-5 in iontophoresis condition #2 (animal 6 data only) mainly due to increasing concentration of GnRH available going from pulses 1-5.

This Example demonstrates pulsatile delivery of GnRH by transdermal iontophoresis both in terms of drug delivery and reproducibility relative to subcutaneous injection. Plasma profiles matching subcutaneous injection were produced for all five dosing cycles.

**Table I: PK parameters (AUC, Cₘₐₓ and Tₘₐₓ) for animal 5 after subcutaneous and iontophoretic delivery of GnRH (condition #2)**

| **Parameter** | **Pulse 1** | **Pulse 2** | **Pulse 3** | **Pulse 4** | **Pulse 5** | **Mean±sd** | **CV** |
|---|---|---|---|---|---|---|---|
| Ionto. AUC (pg-min/mL) | 0 | 0 | 0 | 1451 | 2629 | 2040±832 | 41% |
| | | | | 2160* | 3914 | 3037±1240 | |
| Subcut. AUC (pg-min/mL) | 15038 | 29601 | 37258 | 31952 | 32730 | 29316±8450 | 29% |
| Ionto. Cₘₐₓ (pg/mL) | 0 | 0 | 0 | 67 | 83 | 75±11 | 15% |
| | | | | 101* | 124 | 112±16 | 15% |
| Subcut. Cₘₐₓ (pg/mL) | 379 | 627 | 837 | 712 | 489 | 609±180 | 30% |
| Ionto. Tₘₐₓ (min) | | | | 15 | 15 | 15 | |
| Subcut. Tₘₐₓ (min) | 30 | 15 | 15 | 10 | 21 | 18±8 | 43% |

| | | | | | | | |
|---|---|---|---|---|---|---|---|
| "Ionto" = iontophoresis, "Subcut" = subcutaneous * the second row for Ionto. AUC and Cₘₐₓ is normalized by animal weight | | | | | | | |

**Table J: PK parameters (AUC, Cₘₐₓ and Tₘₐₓ) for animal 6 after subcutaneous and iontophoretic delivery of GnRH (condition #2)**

| **Parameter** | **Pulse 1** | **Pulse 2** | **Pulse 3** | **Pulse 4** | **Pulse 5** | **Mean±sd** | **CV** |
|---|---|---|---|---|---|---|---|
| Ionto. AUC (pg-min/mL) | 0 | 1174 | 2545 | 3032 | 5029 | 2945±1596 | 54% |
| | 0 | 1505* | 3263 | 3887 | 6447 | 3775±2046 | 54% |
| Subcut. AUC (pg-min/mL) | 13923 | 10443 | 13552 | 13431 | 18814 | 14033±3014 | 21% |
| Ionto. Cₘₐₓ (pg/mL) | 0 | 48 | 76 | 106 | 148 | 95±42 | 45% |
| | | 62* | 97 | 136 | 190 | 121±55 | 45% |
| Subcut. Cₘₐₓ (pg/mL) | 353 | 195 | 255 | 211 | 413 | 285±94 | 33% |
| Ionto. Tₘₐₓ (min) | 0 | 20 | 10 | 10 | 10 | 13±5 | 38% |
| Subcut. Tₘₐₓ (min) | 20 | 30 | 20 | 20 | 10 | 20±7 | 35% |

| | | | | | | | |
|---|---|---|---|---|---|---|---|
| "Ionto" = iontophoresis, "Subcut" = subcutaneous * the second row for Ionto. AUC and Cₘₐₓ is normalized by animal weight | | | | | | | |

### Example 2

This Example evaluated the effect of drug concentration and iontophoretic pulse duration on GnRH iontophoretic delivery as well as the reproducibility of drug delivery, using an iontophoretic device according to Patch Fabrication Platform I.

Six prepubescent female Yorkshire pigs (numbered from 7-12) were used in this set of studies. Each animal received two iontophoretic treatments. A first set of three animals received 50 mg/mL drug concentration using five 8-min pulses (condition #3) and a week later received the same concentration using eight 5-min pulses (condition #5). A second set of three animals received 25 mg/mL drug concentration using five 8-min pulses (condition #4) and a week later received the same concentration using eight 5-min pulses (condition #6). The five 8-min pulse experiments were performed first in the sequence. During the span of the study the animal weights ranged from approximately 18-33 kg.

The animals were prepared as described in Example 1. Iontophoretic hydrogel patches prepared according to Patch Fabrication Platform I (5 cm² active area) were used in these studies. The patches were loaded with GnRH (HCl) solution to provide final concentration of either 25 mg/mL GnRH (HCl) or 50 mg/mL GnRH (HCl) in the patch. The pH of the gel surface was measured to be 5.0 ± 0.5. The patches were placed on the back of the animal just off the midline section. The controller, as described in Example 1, was used. One patch each was used through out the entire delivery period for conditions 3-5. For condition #6, the patch was replaced with a fresh patch after pulse 5. The experimental conditions are summarized in Table K. Example 2 utilized current delivery profiles comprising five cycles of eight minute pulses each having a current of 1.2 mA for conditions #3 and #4; and eight cycles of five minute pulses each having a current of 1.2 mA for conditions #5 and #6. Each profile had rest times of 90 minutes between pulses.

**Table K: Experimental Conditions (Example 2)**

| *Iontophoretic Condition* | *Drug Concentration in patch* | *Current* | *# of 90 min cycles- Iontophoretic pulse duration* | *Animal* |
|---|---|---|---|---|
| 3 | 50mg/mL GnRH | 1.2mA | Five-8min | Pig 7, 8, 9 |
| 4 | 25 mg/mL GnRH | 1.2mA | Five-8min | Pig 10, 11, 12 |
| 5 | 50 mg/mL GnRH | 1.2mA | Eight-5min | Pig 7, 8, 9 |
| 6 | 25 mg/mL GnRH | 1.2mA | Eight-5min | Pig 10, 11, 12 |

Blood samples were drawn from a jugular catheter at times as disclosed in Table L, and treated according to the protocol set forth in Example 1.

**Table L: Blood Sample Times (Example 2)**

| Time (min) | Pulse 1 | Pulse 3 | Pulse 4 | Pulse 5 | Pulse 8 |
|---|---|---|---|---|---|
| -15 | X | | | | |
| 0 | X | X . | X | X | X |
| 2 | X (5min pulse duration only) | X (5min pulse duration only) | X (5min pulse duration only) | X (5min pulse duration only) | X (5min pulse duration only) |
| 5 | X | X | X | X | X |
| 10 | X | X | X | X | X |
| 15 | X | X | X | X | X |
| 20 | X | X | X | X | X |
| 30 | X | X | X | X | X |
| 60 | X | X | X | X | X |
| 90 | X | . | | X | X |

| | | | | | |
|---|---|---|---|---|---|
| Five 8-minute pulse samples: total 31 blood samples at 3.0 mL = 93 mL Eight 5-minute pulse samples: total 44 blood samples at 3.0 mL = 132 mL | | | | | |

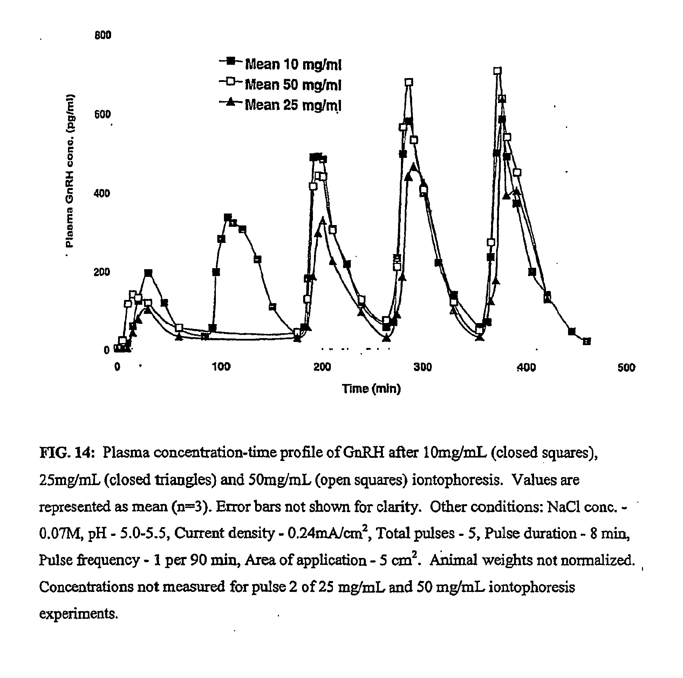

Fig. 14 shows mean plasma concentration-time profiles at three different initial drug concentrations in the patch (10 mg/mL (from Example 1), 25 mg/mL, and 50 mg/mL) using five 8-min pulses (n=3). The general shape of plasma concentration-time profile following iontophoresis (conditions #3 and #4) was again comparable to subcutaneous injection profile (Example 1). Table M shows pharmacokinetic ("PK") parameters (area under the curve ("AUC"), maximum concentration of drug in the bloodstream in a set period of time ("Cₘₐₓ"), and time at which the drug is at the maximum concentration in the bloodstream ("Tₘₐₓ")) obtained after iontophoresis conditions #3 and #4, as well as after condition #1 (10mg/mL iontophoresis) and subcutaneous injection obtained from Example 1. The weight-normalized mean AUC and Cₘₐₓ for 25 and 50 mg/mL concentrations were lower than 10 mg/mL concentration. The results of this Example, like those of Example 1, demonstrate good reproducibility of drug delivery over pulses 3-5 at both 25 and 50 mg/mL concentrations.

**Table M: PK parameters (AUC, Cₘₐₓ and Tₘₐₓ) after five-8min pulses (condition #3, 4, n=3 as well as #1 and subcutaneous from Example 1)**

| **Parameter** | **Pulse 1** | **Pulse 3** | **Pulse 4** | **Pulse 5** | **Mean±sd** |
|---|---|---|---|---|---|
| 10mg/mL AUC (pg-min/mL) | 6941±5158 | 20343±8289 | 17367±7324 | 20474±10571 | 16821±6839 |
| | 9488±6645* | 28178±10949 | 25513±11747 | 30137±16667 | 23329±9419 |
| 25mg/mL AUC (pg-min/mL) | 4886±4555 | 16024±4982 | 25543±6618 | 25528±6939 | 17995±9822 |
| | 3727±3475* | 12253±3809 | 19496±5051 | 19685±5350 | 13790±7548 |
| 50mg/mL AUC pg-min/mL) | 5516±2890 | 19096±6175 | 23819±8949 | 24489±9283 | 18230±8809 |
| | 4148±2173* | 14360±4643 | 17911±6729 | 18415±6980 | 13708±6624 |
| Subcut.AUC (pg-min/mL) | 44519±22292 | 49860±15085 | 30855±9268 | 50532±10389 | 43941±9129 |
| 10mg/mL Cₘₐₓ (pg/mL) | 192±140 | 535±197 | 574±370 | 565±290 | 466±184 |
| | 262±177* | 743±272 | 8.47±575 | 831±458 | 670±276 |
| 25mg/mL Cₘₐₓ (pg/mL.) | 102±112 | 349±65 | 495±117 | 564±275 | 378±204 |
| | 78±85* | 266±50 | 377±89 | 423±206 | 286±153 |
| 50mg/mL Cₘₐₓ (pg/mL) | 148±100 | 450±162 | 604±174 | 717±315 | 480±246 |
| | 110±74 | 330±119 | 445±128 | 530±233 | 354±181 |
| Subcut. Cₘₐₓ | 1074±778 (Pg/mL) | 1036±368 | 6390±229 | 856±114 | 901±199 |
| 10mg/mL Tₘₐₓ (min) | 30±0 | 15±5 | 18±4 | 15±0 | 20±6 |
| 25mg/mL Tₘₐₓ (min) | 38±12 | 30±13 | 32±13 | 27±6 | 32±11 |
| 50mg/mL Tₘₐₓ (min) | 22±8 | 17±3 | 12±3 | 13±6 | 16±6 |
| Subcut Tₘₐₓ (min) | 22±14 | 17±3 | 15±0 | 25±17 | 19±4 |

| | | | | | |
|---|---|---|---|---|---|
| "Ionto" = iontophoresis, "Subcut" = subcutaneous * the second row for Ionto. AUC and C ₘₐₓ is normalized by animal weight (25 kg) to facilitate comparison with 10 mg/mL conc. from the previous study | | | | | |

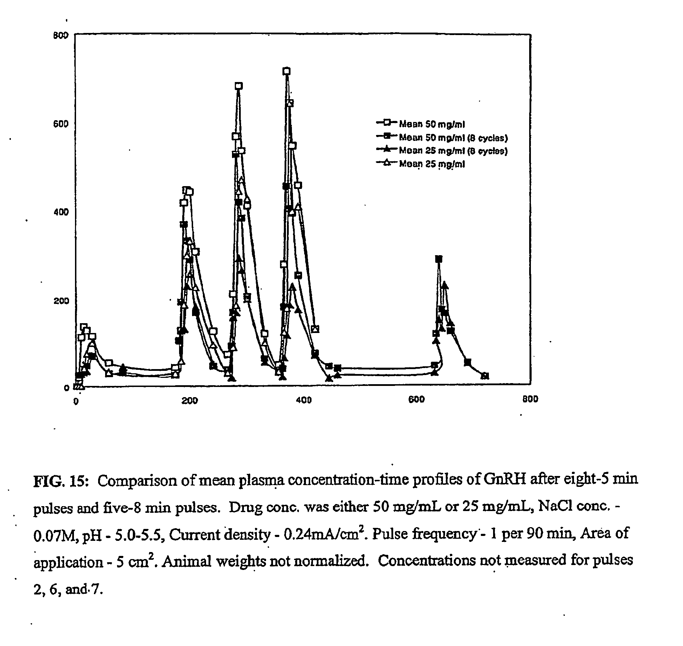

Fig. 15 shows mean plasma concentration-time profiles after iontophoresis conditions #5 and #6 (n=3), conditions #4 and #5 are also shown for comparison. Table N shows PK parameters (AUC, Cₘₐₓ, and Tₘₐₓ) obtained after iontophoresis conditions #5 and #6. With eight 5-min pulses, there is a concentration effect on GnRH delivery. For condition #5 using 50 mg/mL concentration, the mean AUC and Cₘₐₓ were about 2-fold higher than iontophoresis condition #6 using 25 mg/mL concentration, consistent with concentration difference. The mean Tₘₐₓ is 32 ± 11 min and 16 ± 6 min for conditions 3 and 4 versus 20 ± 7 min and 13 ± 5 min for conditions #5 and 6 reflecting relatively sharper rise to Cₘₐₓ for 5-min pulses compared to 8-min pulses. Mean AUC and Cₘₐₓ for 50 mg/mL drug concentration and 5-min pulses were 25% less than those seen for 10 mg/mL drug concentration and 8-min pulses (Example 1). It should be noted that 10 mg/mL drug concentration and 8-min pulses.(Example 1) gave drug delivery which was within acceptable criteria relative to subcutaneous delivery. The data also suggests reproducible drug delivery for pulse 8 relative to pulses 3-5 (only one data point was available for 50 mg/mL group).

**Table N: PK parameters (AUC, Cₘₐₓ and Tₘₐₓ) after eight 5-min pulses (Conditions #5 and #6)**

| **Parameter** | **Pulse 1** | **Pulse 3** | **Pulse 4** | **Pulse 5** | **Pulse 8** | **Mean**±**sd** |
|---|---|---|---|---|---|---|
| 25mg/mL (pg-min/mL) | 1745±300 2094±250* | 5620±1039 6744±1247 | 7798±3230 9358±3876 | 6665±1716 7998±2059 | 5042±164 6050±197 | 5374±2284 6448±2741 |
| 50mg/mL AUC (pg-min/mL) | 3106±357 | 11760±5032 | 15373±7879 | 16642±7649 | 20968±0 | 1356±6711 |
| | 2773±318* | 10500±4492 | 13725±7034 | 14858±6829 | 18721±0 (only one animal) | 12115±5992 |
| 25mg/mL Cₘₐₓ (pg/mL) | 48±0.3 | 204±71 | 197±60 | 151±8 | 164±44 | 152±56 |
| | 58±0.4* | 245±85 | 236±72 | 181±10 | 196±53 | 183±75 |
| 50mg/mL Cₘₐₓ (pg/mL) | 60±22 | 378±147 | 524±377 | 450±211 | 558±0 | 394±199 |
| | 54±20* | 337±131 | 468±336 | 402±214 | 498±0 | 352±178 |
| 25mg/mL Tₘₐₓ (min) | 30±0.0 | 18±4 | 13±4 | 20±0 | 15±7 | 20±7 |
| 50mg/mL Tₘₐₓ (min) | 22±14 | 8±3 | 12±3 | 12±3 | 10±0 | 13±5 |

| | | | | | | |
|---|---|---|---|---|---|---|
| "Ionto" = iontophoresis, "Subcut" = subcutaneous * the second row for Ionto. AUC and Cₘₐₓ are normalized by animal weight (25kg) to facilitate comparison with 10 mg/mL conc. from the previous study | | | | | | |

This Example demonstrates that increasing initial drug concentration in the patch over 10 mg/mL (up to 50 mg/mL) does not facilitate GnRH delivery using five 8-min pulses. However, there was an increase in drug delivery (AUC and Cₘₐₓ) with concentration in the range of 25 to 50 mg/mL using eight 5-min pulses. Furthermore, the data suggests that delivery profiles appear to be relatively sharper for 5-min pulses as compared to 8-min pulses and there is good reproducibility of GnRH delivery over eight 5-min pulses.

Increasing the initial GnRH concentration in the patch from 10 mg/mL to 50 mg/mL did not facilitate GnRH iontophoretic delivery using 0.24 mA/cm² current density and five 8-minute pulses. When eight 5-minute pulses at 0.24 mA/cm² were applied, drug delivery proportionally increased with GnRH concentrations from 25 mg/mL to 50 mg/mL. Five-minute pulses appeared to produce relatively sharper plasma profiles compared to 8-minute pulses. The drug delivery was 25% less when 50 mg/mL drug concentration and 5-minute pulses were applied as compared with 10 mg/mL drug concentration and 8-minute pulses.

### Example 3

This Example assessed the reproducibility of iontophoretic delivery of GnRH using 0.24 mA/cm² current density, 90 minute cycles with eight 5-minute pulses and 25 mg/mL drug concentration (loading concentration of 64.5 mg/mL) in an electrode assembly according to Patch Fabrication Platform I. GnRH delivery over eight five-minute pulses was reproducible.

Three prepubescent female Yorkshire pigs (numbered 13-15) were used in this Example. Each animal received one iontophoretic treatment for eight 90 minute cycles and a ninth cycle with subcutaneous ("SC") dosing for comparison. The ionotophoretic devices were prepared according to Patch Fabrication Platform I (5 cm² active area). The patches were loaded with GnRH (HCI) solution to provide final concentration of 25 mg/mL GnRH. The pH of the gel surface was measured to be 5.0 ± 0.5. The patches were stored at 5°C at all times except during travel to the study site. The SC dosing solution used in animal 13 was prepared 4 days before use and stored at room temperature ("RT"). The SC dosing solution used in animal 14 was prepared 6 days before use and stored mainly at RT. The SC dosing solution used in animal 15 was prepared 1 day before use and stored at RT. During the study, the animal weights ranged from approximately 15-30 kg. The experimental conditions are summarized in Table O. Example 3 utilized a current delivery profile comprising eight cycles of five minute pulses each having a current of 1.2 mA, with rest times of 90 minutes between pulses. **Table O:** Experimental Conditions (Example 3)

| Iontophoretic Condition | Drug Concentration in Patch | Current | # of 90min Cycles-Iontophoretic pulse duration | Animal |
|---|---|---|---|---|
| 6 | 25 mg/mL GnRH | 1.2 mA | Eight 5-min | Pig 13, 14, 15 |

The animals were prepared as described in Example 1. The drug loaded patches were placed on the back of the animal just off the midline section. One patch each was used throughout the eight delivery pulses. The controller, as described in Example 1, was used as a constant current source.

Blood samples were drawn from a jugular catheter at times as disclosed in Table P, and treated according to the analytical protocol set forth in Example 1.

**Table P: Blood Sample Times**

| Time (min) | Pulse 1 5-min pulses | Pulse 2-8 5-min pulses | Pulse 9 SC |
|---|---|---|---|
| -15 | X | | |
| 0 | X | X | X |
| 2 | X | X | X |
| 5 | X | X | X |
| 10 | X | X | X |
| 15 | X | X | X |
| 20 | X | X | X |
| 30 | X | X | X |
| 60 | X | X | X |
| 90 | | | X |

| | | | |
|---|---|---|---|
| Total samples 74 at 3.0 mL = 222 mL of blood volume | | | |

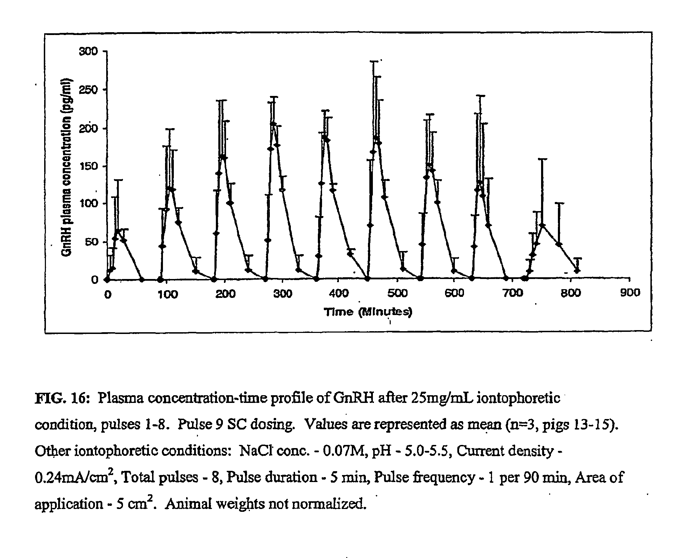

Figure 16 shows mean plasma concentration-time profiles at 25 mg/mL drug concentrations in the patch using eight 5-minute pulses (n=3, pigs 13-15). The general shape of the plasma concentration-time profile following iontophoresis (condition #6) was comparable to subcutaneous injection profile (Example 1). Table Q shows pharmacokinetic parameters (area under the curve ("AUC"), maximum concentration of drug in the bloodstream in a set period of time ("Cₘₐₓ"), and time at which the drug is at the maximum concentration in the bloodstream ('Tₘₐₓ")) obtained after iontophoresis condition #6 during Example 3 and Example 2. The weight-normalized mean AUC and Cₘₐₓ are comparable between the two Examples. Although the GnRH delivery profiles do not match the subcutaneous levels seen in Example 1, these results demonstrate that there is very good reproducibility of drug delivery over pulses 2-8 with AUC, Cₘₐₓ, and Tₘₐₓ, coefficients of variation of 22%, 17%, and 9% respectively for the weight normalized data.

**Table Q: PK parameters (AUC, Cₘₐₓ, and Tₘₐₓ) after eight 5-minute pulses (condition 25 mg/mL drug concentration, Example 3 (n=3, pigs 13, 14, and 15) and Example 2 (n=3, pigs 10,11, and 12)**

| **Parameter** | AUC (pg-min/mL) **Example 3** | AUC (pg-min/mL) **Example 2** | Cₘₐₓ (pg/mL) **Example 3** | Cₘₐₓ (pg/mL) **Example 2** | Tₘₐₓ (min) **Example 3** | Tₘₐₓ (min) **Example 2** |
|---|---|---|---|---|---|---|
| **Pulse 1** | 1910±1102 | 1745±300 | 76±52 | 48±0.3 | 23+6 | 30±0.0 |
| | 1461±894* | 2094±250* | 61±47* | 58±0.4* | | |
| **Pulse 2** | 3934±2171 | NS | 130±62 | NS | 17±3 | NS |
| | 2901±1207* | | 98±43* | | | |
| **Pulse 3** | 5274±2216 | 5620±1039 | 173±58 | 204±71 | 15±5 | 18+4 |
| | 3967±11.90* | 6744±1247* | 1.33±43* | 245+85* | | |
| **Pulse 4** | 6090±1494 | 7798±3230 | 210±36 | 197±60 | 13±3 | 13±4 |
| | 4671±518* | 9358+3876* | 165±37* | 236±72* | | |
| **Pulse 5** | 6331±1021 | 6665±1716 | 187±34 | 151±8 | 15±0.0 | 20±0 |
| | 4910±479* | 7998±2059* | 147±36* | 181±1.0* | | |
| **Pulse 6** | 5897±2517 | NS | 213±90 | NS | 15±5 | NS |
| | 4428±1322* | | 160±50* | | | |
| **Pulse 7**** | 6062±248 | NS | 195±32 | NS | 13±4 | NS |
| | 4446±1227* | | 145±57* | | | |
| **Pulse 8**** | 5398±692 | 5042±164 | 192±42 | 164±44 | 13+4 | 1.5+7 |
| | 4000±1436* | 6050±197* | 144±64* | 196±53* | | |
| **Mean (all sampled pulses 1,3-5,8)** | 5001±1785 | 5374±2284 | 168±53 | 152±56 | 1.6±4 | 20±7 |
| | 3802±1372* | 6448±2741* | 130±40* | 183±75* | | |
| **CV (all sampled pulses 2-8)** | 21% | 19% | 16% | 14% | 9% | 20% |
| | 22% | 25% | 17% | 20% | | |

| | | | | | | |
|---|---|---|---|---|---|---|
| *the second row for Ionto. AUC and Cₘₐₓ is normalized by animal weight (25 kg) to facilitate comparison with previous study **For Study 3, AUC, Cₘₐₓ, and Tₘₐₓ were calculated only for pigs 13 and 15 due to the fact that for pig 14 the patch started to fail in terms of the electrode capacity. NS= Specified pulses were Not Sampled as per the protocol. | | | | | | |

Used patches were extracted to demonstrate the delivery efficiency of the patches used in this Example. The initial concentration prior to use was assayed to be 18.7 mg/patch with a coefficient of variation of 5%. The concentration of the patches after they were used was assayed to be 17.3 mg/patch with a coefficient of variation of 5%. This correlated to a mean recovery of 93% from the used patches.

This Example demonstrated that GnRH delivery at a 25 mg/mL drug concentration, with 5-minute pulse duration has good reproducibility of GnRH delivery profiles for up to eight 90-minute cycles.

Whereas particular embodiments of the invention have been described herein for the purpose of illustrating the invention and not for the purpose of limiting the same, it will be appreciated by those of ordinary skill in the art that numerous variations of the details, materials and arrangement of parts may be made within the principle and scope of the invention without departing from the invention as described in the appended claims. Preferred aspects of the present invention are set out in the following numbered paragraphs of the description.
1. An integrated electrode assembly structured for use in association with an electrically assisted delivery device for delivery of a composition through a membrane, said integrated electrode assembly comprising:
   a flexible backing;
   an electrode layer connected to said flexible backing, said electrode layer having at least a donor electrode and a return electrode;
   at least one lead extending from each of said donor electrode and said return electrode to a tab end portion of said assembly, said tab end portion being structured for electrical connection with at least one component of said electrically assisted delivery device;
   a donor reservoir positioned in electrical communication with said donor electrode, said donor reservoir including an amount of a composition comprising GnRH;
   a return reservoir positioned in electrical communication with said return electrode; and,
   at least one of the following:
      (a) an insulating dielectric coating positioned adjacent to at least a portion of at least one of said electrodes and said leads;
      (b) at least one spline formed in said electrode layer;
      (c) a tab stiffener connected to said tab end portion;
      (d) a tab slit formed in said tab end portion;
      (e) a sensor trace positioned on said tab end portion; (f) a release cover having a donor portion structured to cover said donor reservoir and a return portion structured to cover said return reservoir;
      (g) at least a portion of said flexible backing having a flexural rigidity less than a flexural rigidity of at least a portion of said electrode layer;
      (h) wherein a shortest distance between a surface area of an assembly including said donor electrode and said donor reservoir and a surface area of an assembly including said return electrode and said return reservoir being sized to provide a substantially uniform path of delivery for said composition through said membrane;
      (i) wherein a surface area of an assembly including said donor electrode and said donor reservoir is greater than a surface area of an assembly including said return electrode and said return reservoir;
      (j) wherein a ratio of a surface area of at least one of said reservoirs to a surface area of its corresponding electrode is in the range of about 1.0 to about 1.5;
      (k) wherein a footprint area of said assembly is in the range of about 5 cm² to about 100 cm²;
      (l) wherein a ratio of a total surface area of said electrodes to a total footprint area of said assembly is in the range of about 0.1 to about 0.7;
      (m) wherein a ratio of a surface area of said donor electrode to a surface area of said return electrode is in the range of about 0.1 to about 5.0;
      (n) wherein a ratio of a thickness of said donor reservoir to a thickness of said return reservoir is in the range of about 0.1 to about 2.0;
      (o) wherein at least one component of said assembly in surface contact with at least one of said reservoirs has an aqueous absorption capacity less than an aqueous absorption capacity of said reservoir in surface contact with said component of said assembly;
      (p) a slit formed in said flexible backing in an area located between said donor electrode and said return electrode;
      (q) at least one non-adhesive tab extending from said flexible backing,
      (r) a gap formed between a portion of a layer of transfer adhesive deposited on said electrode layer and a portion of a tab stiffener connected to said tab end portion;
      (s) a tab stiffener attached to a portion of said tab end portion;
      (t) at least one tactile sensation aid formed in said tab end portion;
      (u) at least one indicium formed on at least a portion of said assembly;
      (v) a minimum width of a portion of a layer of transfer adhesive deposited on said electrode layer adjacent to at least one of said donor electrode and said return electrode is in the range of at least about 0.953 cm; and
      (w) a minimum tab length associated with said tab end portion is in the range of at least about 3.8 cm.
2. The assembly of Paragraph 1, wherein said composition comprises from about 5 mg/mL to about 75 mg/mL GnRH concentration in said donor reservoir.
3. The assembly of Paragraph 1, wherein said composition comprises from about 10 mg/L to about 50 mg/L GnRH concentration in said donor reservoir.
4. The assembly of Paragraph 1, wherein at least one of said electrodes comprises a material selected from the group consisting of Ag and Ag/ AgCl.
5. The assembly of Paragraph 1, wherein said donor electrode and said return electrode each have a specific capacity of greater than 1 mA.min/cm².
6. The assembly of paragraph 1, wherein said integrated electrode assembly comprises an insulating dielectric coating positioned adjacent to at least a portion of at least one of said electrodes and said leads.
7. The assembly of Paragraph 6, wherein said dielectric coating is positioned adjacent to at least a portion of a periphery of at least one of said electrodes.
8. The assembly of paragraph 1, wherein said integrated electrode assembly comprises at least one spline formed in said electrode layer.
9. The assembly of paragraph 1, wherein said integrated electrode assembly comprises a tab stiffener connected to said tab end portion.
10. The assembly of paragraph 1, wherein said integrated electrode assembly comprises a tab slit formed in said tab end portion.
11. The assembly of Paragraph 10, further comprising said tab slit being structured to receive a knife edge component of said electrically assisted delivery device.
12. The assembly of Paragraph 11, further comprising said tab slit being structured to be cut by said knife edge upon removal of said tab end portion from said electrically assisted delivery device.
13. The assembly of paragraph 1, wherein said integrated electrode assembly comprises a sensor trace positioned on said tab end portion.
14. The assembly of Paragraph 13, further comprising said sensor trace being structured to permit detection of the presence of said assembly upon electrical association of said assembly with a component of said electrically assisted delivery device.
15. The assembly of paragraph 1, wherein said integrated electrode assembly comprises a release cover having a- donor portion structured to cover said donor reservoir and a return portion structured to cover said return reservoir.
16. The assembly of Paragraph 15, further comprising at least one of said donor portion and said return portion including therein at least one transfer absorbent.
17. The assembly of Paragraph 16, further comprising said transfer absorbent being attached to said release cover with at least one weld.
18. The assembly of Paragraph 17, further comprising said welds being substantially uniformly distributed in an area of connection between said transfer absorbent and said donor portion of said release cover.
19. The assembly of Paragraph 17, further comprising said welds being substantially uniformly distributed in an area of connection between said transfer absorbent and said return portion of said release cover.
20. The assembly of paragraph 1, wherein said integrated electrode assembly comprises at least a portion of said flexible backing having a flexural rigidity less than a flexural rigidity of at least a portion of said electrode layer.
21. The assembly of paragraph 1, wherein a shortest distance between a surface area of an assembly including said donor electrode and said donor reservoir and a surface area of an assembly including said return electrode and said return reservoir being sized to provide a substantially uniform path of delivery for said composition through said membrane.
22. The assembly of Paragraph 21, wherein said shortest distance is in the range of at least about 0.64 cm.
23. The assembly of paragraph 1, wherein a surface area of an assembly including said donor electrode and said donor reservoir is greater than a surface area of an assembly including said return electrode and said return reservoir.
24. The assembly of paragraph 1, wherein a ratio of a surface area of at least one of said reservoirs to a surface area of its corresponding electrode is in the range of about 1.0 to 1.5.
25. The assembly of Paragraph 24, wherein a surface area of at least one of said reservoirs is substantially the same as a surface area of its corresponding electrode.
26. The assembly of paragraph 1, wherein a footprint area of said assembly is in the range of about 5 cm² to 100 cm².
27. The assembly of paragraph 1, wherein a ratio of a total surface area of said electrodes to a total footprint area of said assembly is in the range of about 0.1 to 0.7.
28. The assembly of paragraph 1, wherein a ratio of a surface area of said donor electrode to a surface area of said return electrode is in the range of about 0.1 to 5.0.
29. The assembly of paragraph 1, wherein a ratio of a thickness of said donor reservoir to a thickness of said return reservoir is in the range of about 0.1 to 2.0.
30. The assembly of paragraph 1, wherein at least one component of said assembly in surface contact with at least one of said reservoirs has an aqueous absorption capacity less than an aqueous absorption capacity of said reservoir in surface contact with said component of said assembly.
31. The assembly of paragraph 1, wherein said integrated electrode assembly comprises a slit formed in said flexible backing in an area located between said donor electrode and said return electrode.
32. The assembly of paragraph 1, wherein said integrated electrode assembly comprises at least one non-adhesive tab extending from said flexible backing.
33. The assembly of paragraph 1, wherein said integrated electrode assembly comprises a tab stiffener attached to a portion of said tab end portion; and,
   a gap formed between a portion of said layer of transfer adhesive and said tab stiffener.
34. The assembly of Paragraph 33, wherein a width of said gap is in the range of at least about 1.27 cm.
35. The assembly of paragraph 1, wherein said integrated electrode assembly comprises at least one tactile sensation aid formed in said tab end portion.
36. The assembly of Paragraph 35, wherein said tactile sensation aid includes at least one notch formed in said tab end portion.
37. The assembly of Paragraph 35, wherein said tactile sensation aid includes at least one wing extending from said tab end portion.
38. The assembly of paragraph 1, wherein said integrated electrode assembly comprises at least one indicium formed on at least a portion of said assembly.
39. The assembly of Paragraph 38, wherein said indicium is formed on said flexible backing adjacent to said donor electrode.
40. The assembly of Paragraph 38, wherein said indicium is formed on said flexible backing adjacent to said return electrode.
41. The assembly of paragraph 1, wherein a minimum width of a portion of said layer of transfer adhesive adjacent to at least one of said donor electrode and said return electrode is in the range of at least about 0.953 cm.
42. The assembly of paragraph 1, wherein a minimum tab length associated with said tab end portion is in the range of at least about 3.81 cm.
43. The assembly of Paragraph 1, wherein said composition comprising GnRH comprises one of 10 mg/mL, 25 mg/mL, and 50 mg/mL of GnRH (HCl).
44. The assembly of Paragraph 43, wherein said composition comprising GnRH comprises 25 mg/mL of GnRH (HCI).
45. The assembly of Paragraph 1, wherein said composition comprising GnRH comprises one of 10 mg/mL, 25 mg/mL, and 50 mg/mL of GnRH acetate.
46. The assembly of Paragraph 45, wherein said composition comprising GnRH comprises 25 mg/mL of GnRH acetate.
47. A method of administering a composition through a membrane comprising:
   attaching to the membrane an integrated electrode assembly structured for use in association with an electrically assisted delivery device for delivery of the composition through a membrane, said integrated electrode assembly comprising:
      a flexible backing;
      an electrode layer connected to said flexible backing, said electrode layer having at least a donor electrode and a return electrode;
      at least one lead extending from each of said donor electrode and said return electrode to a tab end portion of said assembly, said tab end portion being structured for electrical connection with at least one component of said electrically assisted delivery device;
      a donor reservoir positioned in electrical communication with said donor electrode, said donor reservoir including an amount of a composition comprising GnRH;
      a return reservoir positioned in electrical communication with said return electrode; and,
      at least one of the following:
         (a) an insulating dielectric coating positioned adjacent to at least a portion of at least one of said electrodes and said leads;
         (b) at least one spline formed in said electrode layer;
         (c) a tab stiffener connected to said tab end portion;
         (d) a tab slit formed in said tab end portion;
         (e) a sensor trace positioned on said tab end portion;
         (f) a release cover having a donor portion structured to cover said donor reservoir and a return portion structured to cover said return reservoir;
         (g) at least a portion of said flexible backing having a flexural rigidity less than a flexural rigidity of at least a portion of said electrode layer;
         (h) wherein a shortest distance between a surface area of an assembly including said donor electrode and said donor reservoir and a surface area of an assembly including said return electrode and said return reservoir being sized to provide a substantially uniform path of delivery for said composition through said membrane;
         (i) wherein a surface area of an assembly including said donor electrode and said donor reservoir is greater than a surface area of an assembly including said return electrode and said return reservoir;
         (j) wherein a ratio of a surface area of at least one of said reservoirs to a surface area of its corresponding electrode is in the range of about 1.0 to 1.5;
         (k) wherein a footprint area of said assembly is in the range of about 5 cm² to 100 cm²;
         (l) wherein a ratio of a total surface area of said electrodes to a total footprint area of said assembly is in the range of about 0.1 to 0.7;
         (m) wherein a ratio of a surface area of said donor electrode to a surface area of said return electrode is in the range of about 0.1 to 5.0;
         (n) wherein a ratio of a thickness of said donor reservoir to a thickness of said return reservoir is in the range of about 0.1 to 2.0;
         (o) wherein at least one component of said assembly in electrical communication with at least one of said reservoirs has an aqueous absorption capacity less than an aqueous absorption capacity of said reservoir in electrical communication with said component of said assembly;
         (p) a slit formed in said flexible backing in an area located between said donor electrode and said return electrode;
         (q) at least one non-adhesive tab extending from said flexible backing;
         (r) a gap formed between a portion of a layer of transfer adhesive deposited on said electrode layer and a portion of a tab stiffener connected to said tab end portion; (s) a tab stiffener attached to a portion of said tab end portion;
         (t) at least one tactile sensation aid formed in said tab end portion;
         (u) at least one indicium formed on at least a portion of said assembly;
         (v) a minimum width of a portion of a layer of transfer adhesive deposited on said electrode layer adjacent to at least one of said donor electrode and said return electrode is in the range of at least about 0.953 cm; and
         (w) a minimum tab length associated with said tab end portion is in the range of at least about 3.81 cm; and
            applying an electrical charge of about 40 mA.min to about 100 mA.min for the electrically assisted delivery device.
48. The method of Paragraph 47, further comprising: applying a pulsatile current delivery profile comprising n pulses, where n is greater than or equal to 1.
49. The method of Paragraph 48, wherein said pulses are unidirectional.
50. The method of Paragraph 49, wherein said pulses comprise rectangular pulses.
51. The method of Paragraph 48, wherein n is greater than 2.
52. The method of Paragraph 48, wherein said pulsatile current delivery profile has a pulse frequency of about 1.0 x 10⁻⁴ Hz to about 5 x 10⁻⁴ Hz.
53. The method of Paragraph 48, wherein said pulses have a rise time of from 0 seconds to about 30 seconds.
54. The method of Paragraph 48, wherein said pulses have a fall time of from 0 seconds to about 30 seconds.
55. The method of Paragraph 48, wherein said pulses have an on-time of about 1 minute to about 15 minutes.
56. The method of Paragraph 55, wherein said pulses have an on-time of about 5 minutes to about 10 minutes.
57. The method of paragraph 48 wherein said pulses have a duty cycle of about 1% to about 20%.
58. The method of paragraph 57, wherein the duty cycle is about 5% to about 10%.
59. The method of paragraph 57, wherein the duty cycle is about 5%.
60. The method of paragraph 48, wherein the number of pulses is 5, the on-time is 8 minutes, the current is 1.2 mA, and the pulse frequency is about 1.85 x 10⁻⁴ Hz.
61. The method of paragraph 48, wherein the number of pulses is 8, the on-time is 5 minutes, the current is 1.2 mA, and the pulse frequency is about 1.85 x 10⁻⁴ Hz.
62. The method of Paragraph 47, wherein said composition comprising GnRH comprises one of 10 mg/mL, 25 mg/mL, and 50 mg/mL of GnRH (HCI).
63. The method of Paragraph 62, wherein said composition comprising GnRH comprises 25 mg/mL of GnRH (HCI).
64. The method of paragraph 47, wherein said composition comprising GnRH comprises one of 10 mg/mL, 25 mg/mL, and 50 mg/mL of GnRH acetate.
65. The method of paragraph 64, wherein said composition comprising GnRH comprises 25 mg/mL of GnRH acetate.

## Claims

1. An integrated electrode assembly structured for use in association with an electrically assisted delivery device for delivery of a composition through a membrane, said integrated electrode assembly comprising:
a flexible backing;
an electrode layer connected to said flexible backing, said electrode layer having at least a donor electrode and a return electrode;
at least one lead extending from each of said donor electrode and said return electrode to a tab end portion of said assembly, said tab end portion being structured for electrical connection with at least on component of said electrically assisted delivery device;
a donor reservoir positioned in electrical communication with said donor electrode, said donor reservoir including an amount of a composition comprising GnRH;
a return reservoir positioned in electrical communication with said return electrode;
at least one spline formed in said electrode layer;
at least a portion of said flexible backing having a flexural rigidity less than a flexural rigidity of at least a portion of said electrode layer;
wherein a ratio of a surface area of at least one of said reservoirs to a surface area of its corresponding electrode is in the range of about 1.0 to about 1.5; and
wherein at least one component of said assembly in surface contact with at least one of said reservoirs has an aqueous absorption capacity less than an aqueous absorption capacity of said reservoir in surface contact with said component of said assembly.

2. The assembly of Claim 1 which further comprises at least one of the following:
1) an insulating dielectric coating positioned adjacent to at least a portion of at least one of said electrodes and said leads;
2) a tab stiffener connected to said tab end portion;
3) a tab slit formed in said tab end portion;
4) a sensor trace positioned on said tab end portion;
5) a release cover having a donor portion structured to cover said donor reservoir and a return portion structured to cover said return reservoir;
6) wherein a shortest distance between a surface area of an assembly including said donor electrode and said donor reservoir and a surface area of an assembly including said return electrode and said return reservoir being sized to provide a substantially uniform path of delivery for said composition through said membrane;
7) wherein a surface area of an assembly including said donor electrode and said donor reservoir is greater than a surface area of an assembly including said return electrode and said return reservoir;
8) wherein a footprint area of said assembly is in the range of about 5 cm² to about 100 cm²;
9) wherein a ratio of a total surface area of said electrodes to a total footprint area of said assembly is in the range of about 0.1 to about 0.7;
10) wherein a ratio of a surface area of said donor electrode to a surface area of said return electrode is in the range of about 0.1 to about 5.0;
11) wherein a ratio of a thickness of said donor reservoir to a thickness of said return reservoir is in the range of about 0.1 to about 2.0;
12) a slit formed in said flexible backing in an area located between said donor electrode and said return electrode;
13) at least one non-adhesive tab extending from said flexible backing,
14) a gap formed between a portion of a layer of transfer adhesive deposited on said electrode layer and a portion of a tab stiffener connected to said tab end portion;
15) a tab stiffener attached to a portion of said tab end portion;
16) at least one tactile sensation aid formed in said tab end portion;
17) at least one indicium formed on at least a portion of said assembly;
18) a minimum width of a portion of a layer of transfer adhesive deposited on said electrode layer adjacent to at least one of said donor electrode and said return electrode is in the range of at least about 0.953 cm; and
19) a minimum tab length associated with said tab end portion is in the range of at least about 3.8 cm.

3. The assembly of Claim 1, wherein said composition comprises from about 5 mg/mL to about 75 mg/mL GnRH concentration in said donor reservoir, preferably from about 10 mg/L to about 50 mg/L GnRH concentration in said donor reservoir.

4. The assembly of Claim 1, wherein at least one of said electrodes comprises a material selected from the group consisting of Ag and Ag/AgCl.

5. The assembly of Claim 1, wherein said donor electrode and said return electrode each have a specific capacity of greater than 1 mA.min/cm².

6. The assembly of Claim 2, wherein said dielectric coating is positioned adjacent to at least a portion of a periphery of at least one of said electrodes.

7. The assembly of Claim 2, further comprising said tab slit being structured to receive a knife edge component of said electrically assisted delivery device, and preferably further comprising said tab slit being structured to be cut by said knife edge upon removal of said tab end portion from said electrically assisted delivery device.

8. The assembly of Claim 2, further comprising said sensor trace being structured to permit detection of the presence of said assembly upon electrical association of said assembly with a component of said electrically assisted delivery device.

9. The assembly of Claim 2, further comprising at least one of said donor portion and said return portion of the release cover including therein at least one transfer absorbent.

10. The assembly of Claim 9, further comprising said transfer absorbent being attached to said release cover with at least one weld, preferably further comprising said welds being substantially uniformly distributed in an area of connection between said transfer absorbent and said donor portion of said release cover, or further comprising said welds being substantially uniformly distributed in an area of connection between said transfer absorbent and said return portion of said release cover.

11. The assembly of Claim 2, wherein said shortest distance is in the range of at least about 0.64 cm.

12. The assembly of Claim 1, wherein a surface area of at least one of said reservoirs is substantially the same as a surface area of its corresponding electrode.

13. The assembly of Claim 2, wherein said tactile sensation aid includes at least one notch formed in said tab end portion or said tactile sensation aid includes at least one wing extending from said tab end portion.

14. The assembly of Claim 1, wherein said composition comprising GnRH comprises one of 10 mg/mL, 25 mg/mL, and 50 mg/mL of GnRH (HCI), preferably 25 mg/mL of GnRH (HCI).

15. The assembly of Claim 1, wherein said composition comprising GnRH comprises one of 10 mg/mL, 25 mg/mL, and 50 mg/mL of GnRH acetate, preferably 25 mg/mL of GnRH acetate.
